# EUROPEAN PATENT APPLICATION

(11) **EP 2 418 491 A1**
(43) Date of publication of application: **15.02.2012**
(21) Application number: 10382229.2
(22) Date of filing: 10.08.2010
(51) Int. Cl.: G01N 33/68

(54) **Method for the diagnosis of dry eye and blepharitis**

(71) Applicant: Bioftalmik, S.L., 48160 Derio (Vizcaya) (ES)
(72) Inventor: Soria Esponera, Javier, 48160 Derio (Bizkaia) (ES); Suarez Cortés, Tatiana, 48160 Derio (Bizkaia) (ES); Gonzalez Fernandez, Nerea, 48160 Derio (Bizkaia) (ES); Acera Osa, Arantxa, 48160 Derio (Bizkaia) (ES)
(74) Representative: ABG Patentes, S.L.

(57) **Abstract**

The invention relates to a method for the diagnosis of dry eyes or blepharitis in a subject which is based on the detection of one or more proteins in a sample from said subject. The invention also relates to a method for differentiating dry eyes and blepharitis.

SEQUENCE LISTING

<110>  Bioftalmik, S.L.

<120>  METHOD FOR THE DIAGNOSIS OF DRY EYE AND BLEPHARITIS

<130>  P5744EP00

<160>  19

<170>  PatentIn version 3.5

<210>  1
<211>  141
<212>  PRT
<213>  Homo sapiens

<400>  1

Met Ala Arg Pro Leu Cys Thr Leu Leu Leu Leu Met Ala Thr Leu Ala
1               5                   10                  15

Gly Ala Leu Ala Ser Ser Ser Lys Glu Glu Asn Arg Ile Ile Pro Gly
            20                  25                  30

Gly Ile Tyr Asp Ala Asp Leu Asn Asp Glu Trp Val Gln Arg Ala Leu
        35                  40                  45

His Phe Ala Ile Ser Glu Tyr Asn Lys Ala Thr Glu Asp Glu Tyr Tyr
    50                  55                  60

Arg Arg Pro Leu Gln Val Leu Arg Ala Arg Glu Gln Thr Phe Gly Gly
65                  70                  75                  80

Val Asn Tyr Phe Phe Asp Val Glu Val Gly Arg Thr Ile Cys Thr Lys
                85                  90                  95

Ser Gln Pro Asn Leu Asp Thr Cys Ala Phe His Glu Gln Pro Glu Leu
            100                 105                 110

Gln Lys Lys Gln Leu Cys Ser Phe Glu Ile Tyr Glu Val Pro Trp Glu
        115                 120                 125

Asp Arg Met Ser Leu Val Asn Ser Arg Cys Gln Glu Ala

130                 135                 140

<210>  2
<211>  141
<212>  PRT
<213>  homo sapiens

<400>  2

Met Ala Gln His Leu Ser Thr Leu Leu Leu Leu Leu Ala Thr Leu Ala
1               5                   10                  15

Val Ala Leu Ala Trp Ser Pro Lys Glu Glu Asp Arg Ile Ile Pro Gly
            20                  25                  30

Gly Ile Tyr Asn Ala Asp Leu Asn Asp Glu Trp Val Gln Arg Ala Leu
        35                  40                  45

His Phe Ala Ile Ser Glu Tyr Asn Lys Ala Thr Lys Asp Asp Tyr Tyr
    50                  55                  60

Arg Arg Pro Leu Arg Val Leu Arg Ala Arg Gln Gln Thr Val Gly Gly
65                  70                  75                  80

Val Asn Tyr Phe Phe Asp Val Glu Val Gly Arg Thr Ile Cys Thr Lys
                85                  90                  95

Ser Gln Pro Asn Leu Asp Thr Cys Ala Phe His Glu Gln Pro Glu Leu
            100                 105                 110

Gln Lys Lys Gln Leu Cys Ser Phe Glu Ile Tyr Glu Val Pro Trp Glu
        115                 120                 125

Asn Arg Arg Ser Leu Val Lys Ser Arg Cys Gln Glu Ser
    130                 135                 140

<210>  3
<211>  90
<212>  PRT
<213>  homo sapiens

<400>  3

Met Ala Cys Pro Leu Asp Gln Ala Ile Gly Leu Leu Val Ala Ile Phe
1               5                   10                  15

His Lys Tyr Ser Gly Arg Glu Gly Asp Lys His Thr Leu Ser Lys Lys
            20                  25                  30

Glu Leu Lys Glu Leu Ile Gln Lys Glu Leu Thr Ile Gly Ser Lys Leu
        35                  40                  45

Gln Asp Ala Glu Ile Ala Arg Leu Met Glu Asp Leu Asp Arg Asn Lys
    50                  55                  60

Asp Gln Glu Val Asn Phe Gln Glu Tyr Val Thr Phe Leu Gly Ala Leu
65                  70                  75                  80

Ala Leu Ile Tyr Asn Glu Ala Leu Lys Gly
                85                  90

<210>  4
<211>  114
<212>  PRT
<213>  homo sapiens

<400>  4

Met Thr Cys Lys Met Ser Gln Leu Glu Arg Asn Ile Glu Thr Ile Ile
1               5                   10                  15

Asn Thr Phe His Gln Tyr Ser Val Lys Leu Gly His Pro Asp Thr Leu
            20                  25                  30

Asn Gln Gly Glu Phe Lys Glu Leu Val Arg Lys Asp Leu Gln Asn Phe
        35                  40                  45

Leu Lys Lys Glu Asn Lys Asn Glu Lys Val Ile Glu His Ile Met Glu
    50                  55                  60

Asp Leu Asp Thr Asn Ala Asp Lys Gln Leu Ser Phe Glu Glu Phe Ile
65                  70                  75                  80

Met Leu Met Ala Arg Leu Thr Trp Ala Ser His Glu Lys Met His Glu
                85                  90                  95

Gly Asp Glu Gly Pro Gly His His His Lys Pro Gly Leu Gly Glu Gly
            100                 105                 110

Thr Pro

<210>  5
<211>  210
<212>  PRT
<213>  homo sapiens

<400>  5

Met Pro Pro Tyr Thr Val Val Tyr Phe Pro Val Arg Gly Arg Cys Ala
1               5                   10                  15

Ala Leu Arg Met Leu Leu Ala Asp Gln Gly Gln Ser Trp Lys Glu Glu
            20                  25                  30

Val Val Thr Val Glu Thr Trp Gln Glu Gly Ser Leu Lys Ala Ser Cys
        35                  40                  45

Leu Tyr Gly Gln Leu Pro Lys Phe Gln Asp Gly Asp Leu Thr Leu Tyr
    50                  55                  60

Gln Ser Asn Thr Ile Leu Arg His Leu Gly Arg Thr Leu Gly Leu Tyr
65                  70                  75                  80

Gly Lys Asp Gln Gln Glu Ala Ala Leu Val Asp Met Val Asn Asp Gly
                85                  90                  95

Val Glu Asp Leu Arg Cys Lys Tyr Ile Ser Leu Ile Tyr Thr Asn Tyr
            100                 105                 110

Glu Ala Gly Lys Asp Asp Tyr Val Lys Ala Leu Pro Gly Gln Leu Lys
        115                 120                 125

Pro Phe Glu Thr Leu Leu Ser Gln Asn Gln Gly Gly Lys Thr Phe Ile

130                 135                 140

Val Gly Asp Gln Ile Ser Phe Ala Asp Tyr Asn Leu Leu Asp Leu Leu
145                 150                 155                 160

Leu Ile His Glu Val Leu Ala Pro Gly Cys Leu Asp Ala Phe Pro Leu
                165                 170                 175

Leu Ser Ala Tyr Val Gly Arg Leu Ser Ala Arg Pro Lys Leu Lys Ala
            180                 185                 190

Phe Leu Ala Ser Pro Glu Tyr Val Asn Leu Pro Ile Asn Gly Asn Gly
        195                 200                 205

Lys Gln
    210

<210>  6
<211>  136
<212>  PRT
<213>  homo sapiens

<400>  6

Met Ser Asn Val Pro His Lys Ser Ser Leu Pro Glu Gly Ile Arg Pro
1               5                   10                  15

Gly Thr Val Leu Arg Ile Arg Gly Leu Val Pro Pro Asn Ala Ser Arg
            20                  25                  30

Phe His Val Asn Leu Leu Cys Gly Glu Glu Gln Gly Ser Asp Ala Ala
        35                  40                  45

Leu His Phe Asn Pro Arg Leu Asp Thr Ser Glu Val Val Phe Asn Ser
    50                  55                  60

Lys Glu Gln Gly Ser Trp Gly Arg Glu Glu Arg Gly Pro Gly Val Pro
65                  70                  75                  80

Phe Gln Arg Gly Gln Pro Phe Glu Val Leu Ile Ile Ala Ser Asp Asp
                85                  90                  95

Gly Phe Lys Ala Val Val Gly Asp Ala Gln Tyr His His Phe Arg His
            100                 105                 110

Arg Leu Pro Leu Ala Arg Val Arg Leu Val Glu Val Gly Gly Asp Val
        115                 120                 125

Gln Leu Asp Ser Val Arg Ile Phe
    130                 135

<210>  7
<211>  346
<212>  PRT
<213>  homo sapiens

<400>  7

Met Ala Met Val Ser Glu Phe Leu Lys Gln Ala Trp Phe Ile Glu Asn
1               5                   10                  15

Glu Glu Gln Glu Tyr Val Gln Thr Val Lys Ser Ser Lys Gly Gly Pro
            20                  25                  30

Gly Ser Ala Val Ser Pro Tyr Pro Thr Phe Asn Pro Ser Ser Asp Val
        35                  40                  45

Ala Ala Leu His Lys Ala Ile Met Val Lys Gly Val Asp Glu Ala Thr
    50                  55                  60

Ile Ile Asp Ile Leu Thr Lys Arg Asn Asn Ala Gln Arg Gln Gln Ile
65                  70                  75                  80

Lys Ala Ala Tyr Leu Gln Glu Thr Gly Lys Pro Leu Asp Glu Thr Leu
                85                  90                  95

Lys Lys Ala Leu Thr Gly His Leu Glu Glu Val Val Leu Ala Leu Leu
            100                 105                 110

Lys Thr Pro Ala Gln Phe Asp Ala Asp Glu Leu Arg Ala Ala Met Lys
        115                 120                 125

Gly Leu Gly Thr Asp Glu Asp Thr Leu Ile Glu Ile Leu Ala Ser Arg
    130                 135                 140

Thr Asn Lys Glu Ile Arg Asp Ile Asn Arg Val Tyr Arg Glu Glu Leu
145                 150                 155                 160

Lys Arg Asp Leu Ala Lys Asp Ile Thr Ser Asp Thr Ser Gly Asp Phe
                165                 170                 175

Arg Asn Ala Leu Leu Ser Leu Ala Lys Gly Asp Arg Ser Glu Asp Phe
            180                 185                 190

Gly Val Asn Glu Asp Leu Ala Asp Ser Asp Ala Arg Ala Leu Tyr Glu
        195                 200                 205

Ala Gly Glu Arg Arg Lys Gly Thr Asp Val Asn Val Phe Asn Thr Ile
    210                 215                 220

Leu Thr Thr Arg Ser Tyr Pro Gln Leu Arg Arg Val Phe Gln Lys Tyr
225                 230                 235                 240

Thr Lys Tyr Ser Lys His Asp Met Asn Lys Val Leu Asp Leu Glu Leu
                245                 250                 255

Lys Gly Asp Ile Glu Lys Cys Leu Thr Ala Ile Val Lys Cys Ala Thr
            260                 265                 270

Ser Lys Pro Ala Phe Phe Ala Glu Lys Leu His Gln Ala Met Lys Gly
        275                 280                 285

Val Gly Thr Arg His Lys Ala Leu Ile Arg Ile Met Val Ser Arg Ser
    290                 295                 300

Glu Ile Asp Met Asn Asp Ile Lys Ala Phe Tyr Gln Lys Met Tyr Gly
305                 310                 315                 320

Ile Ser Leu Cys Gln Ala Ile Leu Asp Glu Thr Lys Gly Asp Tyr Glu
                325                 330                 335

Lys Ile Leu Val Ala Leu Cys Gly Gly Asn
            340                 345

<210>  8
<211>  391
<212>  PRT
<213>  homo sapiens

<400>  8

Met Asp Ser Leu Gly Ala Val Ser Thr Arg Leu Gly Phe Asp Leu Phe
1               5                   10                  15

Lys Glu Leu Lys Lys Thr Asn Asp Gly Asn Ile Phe Phe Ser Pro Val
            20                  25                  30

Gly Ile Leu Thr Ala Ile Gly Met Val Leu Leu Gly Thr Arg Gly Ala
        35                  40                  45

Thr Ala Ser Gln Leu Glu Glu Val Phe His Ser Glu Lys Glu Thr Lys
    50                  55                  60

Ser Ser Arg Ile Lys Ala Glu Glu Lys Glu Val Ile Glu Asn Thr Glu
65                  70                  75                  80

Ala Val His Gln Gln Phe Gln Lys Phe Leu Thr Glu Ile Ser Lys Leu
                85                  90                  95

Thr Asn Asp Tyr Glu Leu Asn Ile Thr Asn Arg Leu Phe Gly Glu Lys
            100                 105                 110

Thr Tyr Leu Phe Leu Gln Lys Tyr Leu Asp Tyr Val Glu Lys Tyr Tyr
        115                 120                 125

His Ala Ser Leu Glu Pro Val Asp Phe Val Asn Ala Ala Asp Glu Ser
    130                 135                 140

Arg Lys Lys Ile Asn Ser Trp Val Glu Ser Lys Thr Asn Glu Lys Ile
145                 150                 155                 160

Lys Asp Leu Phe Pro Asp Gly Ser Ile Ser Ser Ser Thr Lys Leu Val
                165                 170                 175

Leu Val Asn Met Val Tyr Phe Lys Gly Gln Trp Asp Arg Glu Phe Lys
            180                 185                 190

Lys Glu Asn Thr Lys Glu Glu Lys Phe Trp Met Asn Lys Ser Thr Ser
        195                 200                 205

Lys Ser Val Gln Met Met Thr Gln Ser His Ser Phe Ser Phe Thr Phe
    210                 215                 220

Leu Glu Asp Leu Gln Ala Lys Ile Leu Gly Ile Pro Tyr Lys Asn Asn
225                 230                 235                 240

Asp Leu Ser Met Phe Val Leu Leu Pro Asn Asp Ile Asp Gly Leu Glu
                245                 250                 255

Lys Ile Ile Asp Lys Ile Ser Pro Glu Lys Leu Val Glu Trp Thr Ser
            260                 265                 270

Pro Gly His Met Glu Glu Arg Lys Val Asn Leu His Leu Pro Arg Phe
        275                 280                 285

Glu Val Glu Asp Gly Tyr Asp Leu Glu Ala Val Leu Ala Ala Met Gly
    290                 295                 300

Met Gly Asp Ala Phe Ser Glu His Lys Ala Asp Tyr Ser Gly Met Ser
305                 310                 315                 320

Ser Gly Ser Gly Leu Tyr Ala Gln Lys Phe Leu His Ser Ser Phe Val
                325                 330                 335

Ala Val Thr Glu Glu Gly Thr Glu Ala Ala Ala Ala Thr Gly Ile Gly
            340                 345                 350

Phe Thr Val Thr Ser Ala Pro Gly His Glu Asn Val His Cys Asn His
        355                 360                 365

Pro Phe Leu Phe Phe Ile Arg His Asn Glu Ser Asn Ser Ile Leu Phe
    370                 375                 380

Phe Gly Arg Phe Ser Ser Pro
385                 390

<210>  9
<211>  331
<212>  PRT
<213>  homo sapiens

<400>  9

Met Ala Arg Gly Gly Arg Gly Arg Arg Leu Gly Leu Ala Leu Gly Leu
1               5                   10                  15

Leu Leu Ala Leu Val Leu Ala Pro Arg Val Leu Arg Ala Lys Pro Thr
            20                  25                  30

Val Arg Lys Glu Arg Val Val Arg Pro Asp Ser Glu Leu Gly Glu Arg
        35                  40                  45

Pro Pro Glu Asp Asn Gln Ser Phe Gln Tyr Asp His Glu Ala Phe Leu
    50                  55                  60

Gly Lys Glu Asp Ser Lys Thr Phe Asp Gln Leu Thr Pro Asp Glu Ser
65                  70                  75                  80

Lys Glu Arg Leu Gly Lys Ile Val Asp Arg Ile Asp Asn Asp Gly Asp
                85                  90                  95

Gly Phe Val Thr Thr Glu Glu Leu Lys Thr Trp Ile Lys Arg Val Gln
            100                 105                 110

Lys Arg Tyr Ile Phe Asp Asn Val Ala Lys Val Trp Lys Asp Tyr Asp
        115                 120                 125

Arg Asp Lys Asp Asp Lys Ile Ser Trp Glu Glu Tyr Lys Gln Ala Thr
    130                 135                 140

Tyr Gly Tyr Tyr Leu Gly Asn Pro Ala Glu Phe His Asp Ser Ser Asp

145                 150                 155                 160

His His Thr Phe Lys Lys Met Leu Pro Arg Asp Glu Arg Arg Phe Lys
                165                 170                 175

Ala Ala Asp Leu Asn Gly Asp Leu Thr Ala Thr Arg Glu Glu Phe Thr
            180                 185                 190

Ala Phe Leu His Pro Glu Glu Phe Glu His Met Lys Glu Ile Val Val
        195                 200                 205

Leu Glu Thr Leu Glu Asp Ile Asp Lys Asn Gly Asp Gly Phe Val Asp
    210                 215                 220

Gln Asp Glu Tyr Ile Ala Asp Met Phe Ser His Glu Glu Asn Gly Pro
225                 230                 235                 240

Glu Pro Asp Trp Val Leu Ser Glu Arg Glu Gln Phe Asn Glu Phe Arg
                245                 250                 255

Asp Leu Asn Lys Asp Gly Lys Leu Asp Lys Asp Glu Ile Arg His Trp
            260                 265                 270

Ile Leu Pro Gln Asp Tyr Asp His Ala Gln Ala Glu Ala Arg His Leu
        275                 280                 285

Val Tyr Glu Ser Asp Lys Asn Lys Asp Glu Lys Leu Thr Lys Glu Glu
    290                 295                 300

Ile Leu Glu Asn Trp Asn Met Phe Val Gly Ser Gln Ala Thr Asn Tyr
305                 310                 315                 320

Gly Glu Asp Leu Thr Lys Asn His Asp Glu Leu
                325                 330

<210>  10
<211>  222
<212>  PRT
<213>  homo sapiens

<400>  10

<210>  11
<211>  425
<212>  PRT
<213>  homo sapiens

<400>  11

<210>  12
<211>  427
<212>  PRT
<213>  homo sapiens

<400>  12

Met Ser Arg Ser Lys Arg Asp Asn Asn Phe Tyr Ser Val Glu Ile Gly
1               5                   10                  15

Asp Ser Thr Phe Thr Val Leu Lys Arg Tyr Gln Asn Leu Lys Pro Ile
            20                  25                  30

Gly Ser Gly Ala Gln Gly Ile Val Cys Ala Ala Tyr Asp Ala Ile Leu
        35                  40                  45

Glu Arg Asn Val Ala Ile Lys Lys Leu Ser Arg Pro Phe Gln Asn Gln
    50                  55                  60

Thr His Ala Lys Arg Ala Tyr Arg Glu Leu Val Leu Met Lys Cys Val
65                  70                  75                  80

Asn His Lys Asn Ile Ile Gly Leu Leu Asn Val Phe Thr Pro Gln Lys
                85                  90                  95

Ser Leu Glu Glu Phe Gln Asp Val Tyr Ile Val Met Glu Leu Met Asp
            100                 105                 110

Ala Asn Leu Cys Gln Val Ile Gln Met Glu Leu Asp His Glu Arg Met
        115                 120                 125

Ser Tyr Leu Leu Tyr Gln Met Leu Cys Gly Ile Lys His Leu His Ser
    130                 135                 140

Ala Gly Ile Ile His Arg Asp Leu Lys Pro Ser Asn Ile Val Val Lys
145                 150                 155                 160

Ser Asp Cys Thr Leu Lys Ile Leu Asp Phe Gly Leu Ala Arg Thr Ala
                165                 170                 175

Gly Thr Ser Phe Met Met Thr Pro Tyr Val Val Thr Arg Tyr Tyr Arg
            180                 185                 190

Ala Pro Glu Val Ile Leu Gly Met Gly Tyr Lys Glu Asn Val Asp Leu
        195                 200                 205

Trp Ser Val Gly Cys Ile Met Gly Glu Met Val Cys His Lys Ile Leu
    210                 215                 220

Phe Pro Gly Arg Asp Tyr Ile Asp Gln Trp Asn Lys Val Ile Glu Gln
225                 230                 235                 240

Leu Gly Thr Pro Cys Pro Glu Phe Met Lys Lys Leu Gln Pro Thr Val
                245                 250                 255

Arg Thr Tyr Val Glu Asn Arg Pro Lys Tyr Ala Gly Tyr Ser Phe Glu
            260                 265                 270

Lys Leu Phe Pro Asp Val Leu Phe Pro Ala Asp Ser Glu His Asn Lys
        275                 280                 285

Leu Lys Ala Ser Gln Ala Arg Asp Leu Leu Ser Lys Met Leu Val Ile
    290                 295                 300

Asp Ala Ser Lys Arg Ile Ser Val Asp Glu Ala Leu Gln His Pro Tyr
305                 310                 315                 320

Ile Asn Val Trp Tyr Asp Pro Ser Glu Ala Glu Ala Pro Pro Pro Lys
                325                 330                 335

Ile Pro Asp Lys Gln Leu Asp Glu Arg Glu His Thr Ile Glu Glu Trp
            340                 345                 350

Lys Glu Leu Ile Tyr Lys Glu Val Met Asp Leu Glu Glu Arg Thr Lys
        355                 360                 365

Asn Gly Val Ile Arg Gly Gln Pro Ser Pro Leu Gly Ala Ala Val Ile
    370                 375                 380

Asn Gly Ser Gln His Pro Ser Ser Ser Ser Ser Val Asn Asp Val Ser
385                 390                 395                 400

Ser Met Ser Thr Asp Pro Thr Leu Ala Ser Asp Thr Asp Ser Ser Leu
                405                 410                 415

Glu Ala Ala Ala Gly Pro Leu Gly Cys Cys Arg
            420                 425

<210>  13
<211>  795
<212>  PRT
<213>  homo sapiens

<400>  13

<210>  14
<211>  218
<212>  PRT
<213>  homo sapiens

<400>  14

Met Pro Met Thr Leu Gly Tyr Trp Asn Ile Arg Gly Leu Ala His Ser
1               5                   10                  15

Ile Arg Leu Leu Leu Glu Tyr Thr Asp Ser Ser Tyr Glu Glu Lys Lys
            20                  25                  30

Tyr Thr Met Gly Asp Ala Pro Asp Tyr Asp Arg Ser Gln Trp Leu Asn
        35                  40                  45

Glu Lys Phe Lys Leu Gly Leu Asp Phe Pro Asn Leu Pro Tyr Leu Ile
    50                  55                  60

Asp Gly Thr His Lys Ile Thr Gln Ser Asn Ala Ile Leu Arg Tyr Ile
65                  70                  75                  80

Ala Arg Lys His Asn Leu Cys Gly Glu Ser Glu Lys Glu Gln Ile Arg
                85                  90                  95

Glu Asp Ile Leu Glu Asn Gln Phe Met Asp Ser Arg Met Gln Leu Ala
            100                 105                 110

Lys Leu Cys Tyr Asp Pro Asp Phe Glu Lys Leu Lys Pro Glu Tyr Leu
        115                 120                 125

Gln Ala Leu Pro Glu Met Leu Lys Leu Tyr Ser Gln Phe Leu Gly Lys
    130                 135                 140

Gln Pro Trp Phe Leu Gly Asp Lys Ile Thr Phe Val Asp Phe Ile Ala
145                 150                 155                 160

Tyr Asp Val Leu Glu Arg Asn Gln Val Phe Glu Pro Ser Cys Leu Asp
                165                 170                 175

Ala Phe Pro Asn Leu Lys Asp Phe Ile Ser Arg Phe Glu Gly Leu Glu
            180                 185                 190

Lys Ile Ser Ala Tyr Met Lys Ser Ser Arg Phe Leu Pro Arg Pro Val
        195                 200                 205

Phe Thr Lys Met Ala Val Trp Gly Asn Lys
    210                 215

<210>  15
<211>  222
<212>  PRT
<213>  homo sapiens

<400>  15

Met Ala Ala Arg Pro Lys Leu His Tyr Pro Asn Gly Arg Gly Arg Met
1               5                   10                  15

Glu Ser Val Arg Trp Val Leu Ala Ala Ala Gly Val Glu Phe Asp Glu
            20                  25                  30

Glu Phe Leu Glu Thr Lys Glu Gln Leu Tyr Lys Leu Gln Asp Gly Asn
        35                  40                  45

His Leu Leu Phe Gln Gln Val Pro Met Val Glu Ile Asp Gly Met Lys
    50                  55                  60

Leu Val Gln Thr Arg Ser Ile Leu His Tyr Ile Ala Asp Lys His Asn
65                  70                  75                  80

Leu Phe Gly Lys Asn Leu Lys Glu Arg Thr Leu Ile Asp Met Tyr Val
                85                  90                  95

Glu Gly Thr Leu Asp Leu Leu Glu Leu Leu Ile Met His Pro Phe Leu
            100                 105                 110

Lys Pro Asp Asp Gln Gln Lys Glu Val Val Asn Met Ala Gln Lys Ala
        115                 120                 125

Ile Ile Arg Tyr Phe Pro Val Phe Glu Lys Ile Leu Arg Gly His Gly
    130                 135                 140

Gln Ser Phe Leu Val Gly Asn Gln Leu Ser Leu Ala Asp Val Ile Leu
145                 150                 155                 160

Leu Gln Thr Ile Leu Ala Leu Glu Glu Lys Ile Pro Asn Ile Leu Ser
                165                 170                 175

Ala Phe Pro Phe Leu Gln Glu Tyr Thr Val Lys Leu Ser Asn Ile Pro
            180                 185                 190

Thr Ile Lys Arg Phe Leu Glu Pro Gly Ser Lys Lys Lys Pro Pro Pro
        195                 200                 205

Asp Glu Ile Tyr Val Arg Thr Val Tyr Asn Ile Phe Arg Pro
    210                 215                 220

<210>  16
<211>  408
<212>  PRT

<213>  homo sapiens

<400>  16

Met Ala Glu Asn Gly Asp Asn Glu Lys Met Ala Ala Leu Glu Ala Lys
1               5                   10                  15

Ile Cys His Gln Ile Glu Tyr Tyr Phe Gly Asp Phe Asn Leu Pro Arg
            20                  25                  30

Asp Lys Phe Leu Lys Glu Gln Ile Lys Leu Asp Glu Gly Trp Val Pro
        35                  40                  45

Leu Glu Ile Met Ile Lys Phe Asn Arg Leu Asn Arg Leu Thr Thr Asp
    50                  55                  60

Phe Asn Val Ile Val Glu Ala Leu Ser Lys Ser Lys Ala Glu Leu Met
65                  70                  75                  80

Glu Ile Ser Glu Asp Lys Thr Lys Ile Arg Arg Ser Pro Ser Lys Pro
                85                  90                  95

Leu Pro Glu Val Thr Asp Glu Tyr Lys Asn Asp Val Lys Asn Arg Ser
            100                 105                 110

Val Tyr Ile Lys Gly Phe Pro Thr Asp Ala Thr Leu Asp Asp Ile Lys
        115                 120                 125

Glu Trp Leu Glu Asp Lys Gly Gln Val Leu Asn Ile Gln Met Arg Arg
    130                 135                 140

Thr Leu His Lys Ala Phe Lys Gly Ser Ile Phe Val Val Phe Asp Ser
145                 150                 155                 160

Ile Glu Ser Ala Lys Lys Phe Val Glu Thr Pro Gly Gln Lys Tyr Lys
                165                 170                 175

Glu Thr Asp Leu Leu Ile Leu Phe Lys Asp Asp Tyr Phe Ala Lys Lys
            180                 185                 190

Asn Glu Glu Arg Lys Gln Asn Lys Val Glu Ala Lys Leu Arg Ala Lys
        195                 200                 205

Gln Glu Gln Glu Ala Lys Gln Lys Leu Glu Glu Asp Ala Glu Met Lys
    210                 215                 220

Ser Leu Glu Glu Lys Ile Gly Cys Leu Leu Lys Phe Ser Gly Asp Leu
225                 230                 235                 240

Asp Asp Gln Thr Cys Arg Glu Asp Leu His Ile Leu Phe Ser Asn His
                245                 250                 255

Gly Glu Ile Lys Trp Ile Asp Phe Val Arg Gly Ala Lys Glu Gly Ile
            260                 265                 270

Ile Leu Phe Lys Glu Lys Ala Lys Glu Ala Leu Gly Lys Ala Lys Asp
        275                 280                 285

Ala Asn Asn Gly Asn Leu Gln Leu Arg Asn Lys Glu Val Thr Trp Glu
    290                 295                 300

Val Leu Glu Gly Glu Val Glu Lys Glu Ala Leu Lys Lys Ile Ile Glu
305                 310                 315                 320

Asp Gln Gln Glu Ser Leu Asn Lys Trp Lys Ser Lys Gly Arg Arg Phe
                325                 330                 335

Lys Gly Lys Gly Lys Gly Asn Lys Ala Ala Gln Pro Gly Ser Gly Lys
            340                 345                 350

Gly Lys Val Gln Phe Gln Gly Lys Lys Thr Lys Phe Ala Ser Asp Asp
        355                 360                 365

Glu His Asp Glu His Asp Glu Asn Gly Ala Thr Gly Pro Val Lys Arg
    370                 375                 380

Ala Arg Glu Glu Thr Asp Lys Glu Glu Pro Ala Ser Lys Gln Gln Lys
385                 390                 395                 400

Thr Glu Asn Gly Ala Gly Asp Gln
                405

<210>  17
<211>  505
<212>  PRT
<213>  homo sapiens

<400>  17

<210>  18
<211>  90
<212>  PRT
<213>  homo sapiens

<400>  18

Met Arg Leu Ser Val Cys Leu Leu Leu Leu Thr Leu Ala Leu Cys Cys
1               5                   10                  15

Tyr Arg Ala Asn Ala Val Val Cys Gln Ala Leu Gly Ser Glu Ile Thr
            20                  25                  30

Gly Phe Leu Leu Ala Gly Lys Pro Val Phe Lys Phe Gln Leu Ala Lys
        35                  40                  45

Phe Lys Ala Pro Leu Glu Ala Val Ala Ala Lys Met Glu Val Lys Lys
    50                  55                  60

Cys Val Asp Thr Met Ala Tyr Glu Lys Arg Val Leu Ile Thr Lys Thr
65                  70                  75                  80

Leu Gly Lys Ile Ala Glu Lys Cys Asp Arg
                85                  90

<210>  19
<211>  214
<212>  PRT
<213>  homo sapiens

<400>  19

Met Gly Leu Ala Gly Val Cys Ala Leu Arg Arg Ser Ala Gly Tyr Ile
1               5                   10                  15

Leu Val Gly Gly Ala Gly Gly Gln Ser Ala Ala Ala Ala Ala Arg Arg
            20                  25                  30

Cys Ser Glu Gly Glu Trp Ala Ser Gly Gly Val Arg Ser Phe Ser Arg
        35                  40                  45

Ala Ala Ala Ala Met Ala Pro Ile Lys Val Gly Asp Ala Ile Pro Ala
    50                  55                  60

Val Glu Val Phe Glu Gly Glu Pro Gly Asn Lys Val Asn Leu Ala Glu
65                  70                  75                  80

Leu Phe Lys Gly Lys Lys Gly Val Leu Phe Gly Val Pro Gly Ala Phe
                85                  90                  95

Thr Pro Gly Cys Ser Lys Thr His Leu Pro Gly Phe Val Glu Gln Ala
            100                 105                 110

Glu Ala Leu Lys Ala Lys Gly Val Gln Val Val Ala Cys Leu Ser Val
        115                 120                 125

Asn Asp Ala Phe Val Thr Gly Glu Trp Gly Arg Ala His Lys Ala Glu
    130                 135                 140

Gly Lys Val Arg Leu Leu Ala Asp Pro Thr Gly Ala Phe Gly Lys Glu
145                 150                 155                 160

Thr Asp Leu Leu Leu Asp Asp Ser Leu Val Ser Ile Phe Gly Asn Arg
                165                 170                 175

Arg Leu Lys Arg Phe Ser Met Val Val Gln Asp Gly Ile Val Lys Ala
            180                 185                 190

Leu Asn Val Glu Pro Asp Gly Thr Gly Leu Thr Cys Ser Leu Ala Pro
        195                 200                 205

Asn Ile Ile Ser Gln Leu
    210

## Description

### Field of the Invention

The invention is comprised within the field of the disease diagnosis area; specifically, in the development of a method for the diagnosis of dry eye and blepharitis in a subject, based on the detection of determined markers.

### Background of the Invention

The dry eye syndrome (also known as keratitis sicca) is an ocular surface disease which may be due to a reduction of the activity of the lacrimal glands with the consequent lower tear production, in which case it is referred to as "hyposecretory dry eyes" or to an excessive loss of water of the exposed ocular surface in the presence of a normal secretory function, in which case it is known as "evaporative dry eyes".

It is currently defined as a multifactorial disease resulting in ocular discomfort, visual alteration and instability of the tear film, with potential damages of the ocular surface. It is accompanied by an increased osmolarity of the tear film and by inflammation of the ocular surface (DEWS, The Ocular Surface, April 2007, Vol. 5, 2: 69-202).

There are multiple causes which can cause dry eyes, being more common in the elderly. It can be caused, among other factors, by the aging process, use of contact lenses, hormonal changes in women, environmental factors, side effects of diseases/medicinal products, laser surgeries for vision correction, destabilization of the tear composition and chronic eye diseases. The diseases causing dry eyes include vitamin A deficiency, Sjögren's syndrome, rheumatoid arthritis and other rheumatologic diseases, it can also occur due to chemical or thermal burns, and drugs such as atenolol, chlorpheniramine, hydrochlorothiazide, isotretinoin, ketorolac, ketotifen, levocabastine, levofloxacin, oxybutynin or tolterodine. As the disease progresses, there is a thickening of the cornea and a reduction of visual acuity. Other symptoms of dry eyes are a stinging or burning sensation in the eye, foreign body sensation, itching or pruritus, rheum and conjunctival reddening.

Blepharitis is a term which is used to describe the inflammation of the tissue forming the eyelid. Its origin is often due to a malfunctioning of the glands which are located in the eyelid margin. Under normal conditions, these glands produce an oily secretion which aids in lubricating the surface of the eye and the inner side of the eyelids, preventing the evaporation of tears. In subjects with blepharitis, these glands are obstructed, their secretions are stagnant and fatty acids are formed which irritate the ocular surface. The margin of the eyelids is inflamed and reddened in these cases. The eye is irritated and produces secretion of mucus and proteins, and the latter accumulate in the eyelid margin, often creating a crust. The accumulation of these materials provides the optimal conditions for the growth of bacteria, which in turn release toxins which contribute to irritating the eyelids even more and to further aggravating the pathological process. Therefore, in blepharitis there is a chain of events including eyelid gland dysfunction, irritation and formation of small crusts in the eyelid margin, in addition to bacterial infection. The severity of blepharitis varies considerably among individuals. In some cases, it only represents a moderate discomfort, creating a slight, intermittent irritation. In others, it is a more serious disease which can affect vision.

Blepharitis is a common process affecting 5% of the population, with a chronic nature and which is presented in outbreaks. This disease occurs both in men and in women without distinction, but it has a slightly greater incidence in men. Nevertheless, associated with other diseases, it can have an incidence of up to 15% as in the case of ocular cicatricial pemphigoid or of up to 19% in the case of its association with dry eyes. There are two types of blepharitis: anterior (seborrheic or staphylococcal) blepharitis and posterior (hypersecretory or obstructive) blepharitis, the latter occurs due to a meibomian gland dysfunction (MGD). In seborrheic anterior blepharitis, the appearance of the edge of the eyelid is very "oily", with soft and "sticky" plaques or flakes, with abundant yellowish-white secretion at the exit of the glands, with the eyelashes adhered to one another by the oil. Favored by this alteration of glandular secretion, there is bacterial colonization, the most important bacterium causing the infection being a staphylococcus. There is greater reddening due to the direct irritation of the bacteria and their toxins. In staphylococcal posterior blepharitis, there are no oily and sticky flakes as in seborrheic blepharitis, but rather dry, larger crusts which are visible without a microscope. Genuine collarettes are often formed the eyelashes, and it seems like "dandruff" in the eyelashes, with reddened skin and irritated eyes. Posterior blepharitis occurs when the meibomian glands are affected, the inner part of the eyelashes which is in contact with the eye also being affected. This blepharitis has other names, such as meibomitis, meibomian gland dysfunction (MGD), meibomian foam, etc., and although the symptoms do not seem to be as significant as seborrheic anterior blepharitis, it is easier for the ocular surface to be altered. Due to the fact that the meibomian glands are in charge of producing the lipid component of tears, if meibomitis occurs, this component is altered and the tears are "of a poor quality", they break and do not remain homogeneously distributed over the surface of the eye. Thus, when blepharitis is referred to as the cause of dry eyes, reference is almost always made to posterior blepharitis (a meibomian gland problem). Likewise, anterior and posterior blepharitis can occur simultaneously, in fact it is quite usual. For example, in the case of seborrheic blepharitis, there is an alteration of the glands both in the front part (eyelashes "covered" in oil) and in the rear part (meibomitis).

The diagnosis of dry eyes is normally performed with Schirmer's test, which consists of placing a strip of blotting paper hanging from the lower eyelid, keeping the eyes closed for 5 minutes and observing what length of the paper is wet with tears, the normal length being 15 mm. This test has the drawback that tears can be generated due to the irritation with the paper and it is often necessary to use local anesthesia, the test then being referred to as the Jones test. Therefore, several new tests have been developed over time, such as the measurement of lactoferrin, since it seems that the amount of this molecule is closely related to tear production. Patients with low tear production and dry eyes have low levels of lactoferrin. There is also another method which consists of measuring the lysozyme concentration in tears. Another test consists of adding drops of fluorescein in the eye of the patient, such that the fluorescein should pass from the tear duct to the nose in 2 minutes. If the patient does not have enough tears to entrain the marker, this time will be longer.

In relation to the diagnosis of blepharitis, an evaluation of the eyelids during an eye examination is generally enough to diagnose said disease. Another possible way to diagnose blepharitis consists of analyzing a sample from the eye of the patient and checking if it has any bacterial infection and even possible allergies which may be causing blepharitis.

Both diseases, dry eye syndrome and blepharitis, are closely related and often occur together in the patient (being referred to as mixed dry eyes-blepharitis pathology). It is known that approximately 70% of the patients with dry eyes have associated blepharitis (Lemp MA. Report of the National Eye Institute/Industry workshop on Clinical Trials in Dry Eyes. CLAO J. 1995; 21:221-32). It is therefore important to differentiate if the patient suffers only from dry eye syndrome, blepharitis or both pathologies.

It is therefore necessary to develop methods for the diagnosis of dry eye and blepharitis and a method with which both syndromes can be differentiated and which overcomes the mentioned drawbacks; it would be particularly desirable for said method to be simple and sufficiently sensitive for differentiating between both pathologies.

### Summary of the Invention

The inventors have found several markers which can diagnose dry eye syndrome and blepharitis in a sample from a subject, specifically in a tear sample. It has also been demonstrated that both pathologies can be differentiated by means of the detection of said markers. The assays conducted by the inventors have shown that the detection of the markers, for example, from two-dimensional electrophoresis (Example 2), allows differentiating between both pathologies.

Therefore, in one aspect, the invention relates to an in vitro method for diagnosing dry eye in a subject which comprises:
(i) determining the level of one or more proteins selected from the group of Lipophilin A, Peroxiredoxin 5, Cystatin S, Cystatin N, S100A4, S100A6, GST P1, Annexin 1 and Galectin 7 or a functionally equivalent variant thereof, in a sample from a subject; and
(ii) comparing the level of said protein or proteins with the level of said protein or proteins in a reference sample,
   wherein if the subject has dry eye, the level of at least one of the S100A4, S100A6, GST P1 and Annexin 1 proteins is elevated with respect to its level in the reference sample and/or the level of at least one of the Cystatin S, Cystatin N, Galectin 7, Lipophilin A and Peroxiredoxin 5 proteins is reduced with respect to its level in the reference sample.

In another aspect, the invention relates to an *in vitro* method for diagnosing blepharitis in a subject which comprises:
(i) determining the level of one or more proteins selected from the group consisting of Lipophilin A, Peroxiredoxin 5, Cystatin S, Cystatin N, S100A4, S100A6, GST P1, Annexin 1 and Galectin 7 or a functionally equivalent variant thereof, in a sample from a subject; and
(ii) comparing the level of said protein or proteins with the level of said protein or proteins in a reference sample,
   wherein if the subject has blepharitis, the level of at least one of the GST P1 and Annexin 1 proteins is elevated with respect to its level in the reference sample, the level of the S100A4 protein remains identical with respect to its level in the reference sample and/or the level of at least one of the Cystatin S, Cystatin N, S100A6, Galectin 7, Lipophilin A and Peroxiredoxin 5 proteins is reduced with respect to its level in the reference sample.

In another aspect, the invention relates to an *in vitro* method for differentiating between dry eye and blepharitis in a subject which comprises:
(i) determining the level of one or more proteins selected from the group of Lipophilin A, Peroxiredoxin 5, Cystatin S, Cystatin N, S100A4, S100A6, GST P1, Annexin 1 and Galectin 7 or a functionally equivalent variant thereof, in a sample from a subject; and
(ii) comparing the level of said protein or proteins with the level of said protein or proteins in a reference sample,
   wherein if the subject has dry eye, the level of at least one of the GSTP1, S100A6 and S100A4 proteins is elevated with respect to its level in the blepharitis sample and in the reference sample.

In another aspect, the invention relates to an *in vitro* method for diagnosing blepharitis or dry eye in a tear sample from a subject which comprises:
(i) determining the level of Galectin 7 or a functionally equivalent variant thereof in said sample; and
(ii) comparing the level of said protein with its level in a reference sample.

In a final aspect, the invention relates to a kit comprising at least one reagent for the detection of at least one protein selected from the group of Lipophilin A, Peroxiredoxin 5, Cystatin S, Cystatin N, S100A4, S100A6, GST P1, Annexin 1 and Galectin 7 or a functional variant thereof.

### Brief Description of the Drawings

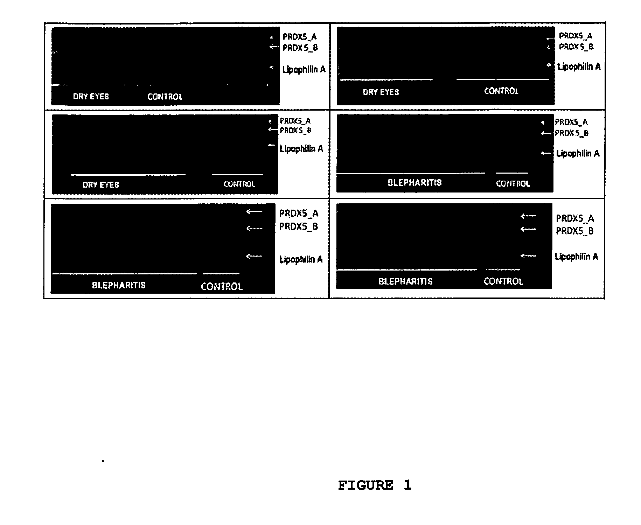

Figure 1 shows the validation by means of Immunofluorescent Multiplex Western Blot of Lipophilin A (lower band) and Peroxiredoxin 5 (upper bands, PRDX5 A and B), two of the candidate markers obtained by means of a literature search. In each case, the group of patients to which it corresponds is indicated.

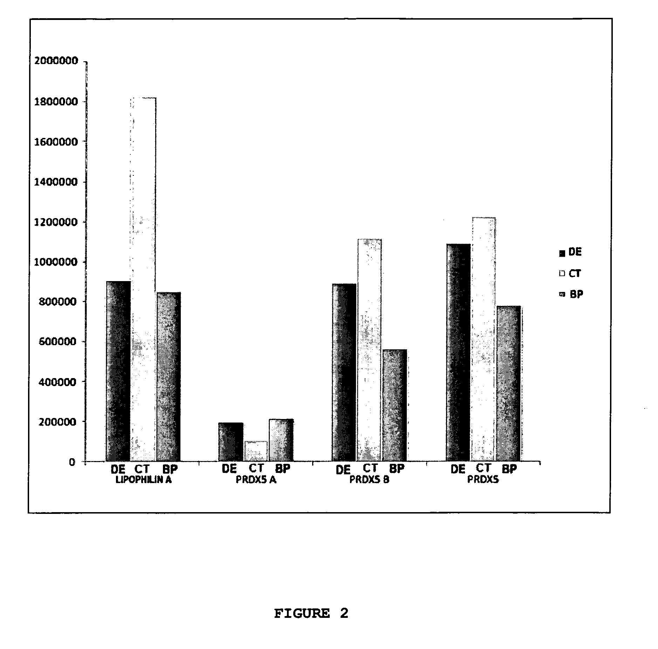

Figure 2 depicts a bar graph representing the normalized volume values for each group and for each marker. A reduction in the Lipophilin A concentration, both in dry eyes (DE) and in blepharitis (BP) with respect to the control (CT), can be observed. In the case of Peroxiredoxin 5, both in dry eyes and in blepharitis, the presence of an isoform (PRDX5A) which was virtually not present in the control group is observed.

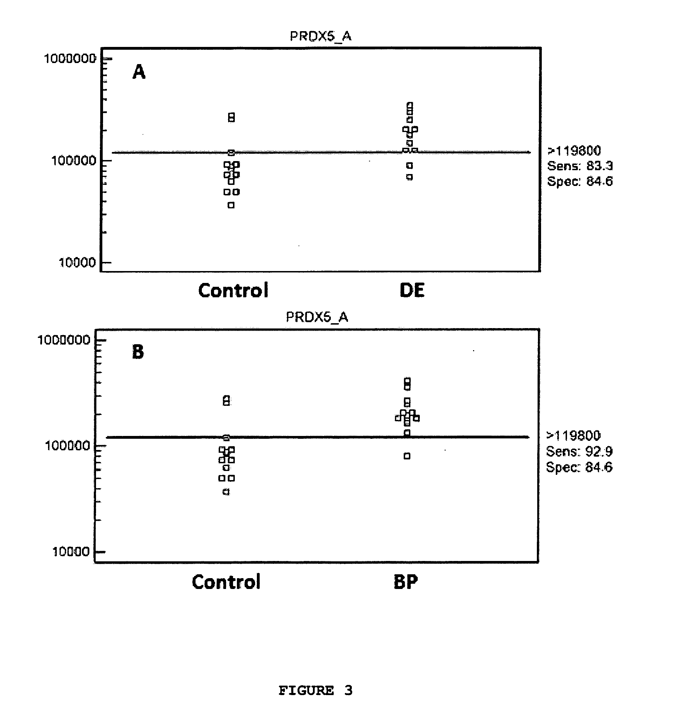

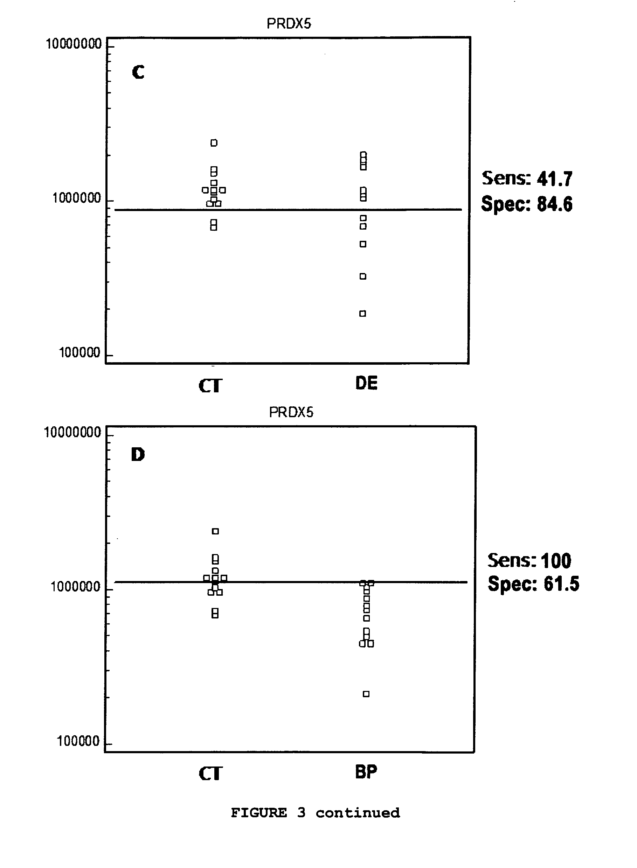

Figure 3 corresponds to a dot plot at a logarithmic scale which depicts the normalized volume values obtained for Peroxiredoxin 5A and Peroxiredoxin 5 (isoforms A+B) by means of Western blot. These graphs comparatively show dry eyes (DE) compared to a control (A) and blepharitis (BP) compared to a control (B) for the Peroxiredoxin 5A isoform. They also comparatively show dry eyes (DE) compared to a control (C) and blepharitis (BP) compared to a control (D) for Peroxiredoxin 5 (both isoforms). The x-axis shows, in the form of logical values, the pathological condition (independent variable) of the samples, whereas the y-axis shows the normalized volume values of said samples. The sensitivity and specificity values are indicated at the right of the plots (Sens and Spec, respectively).

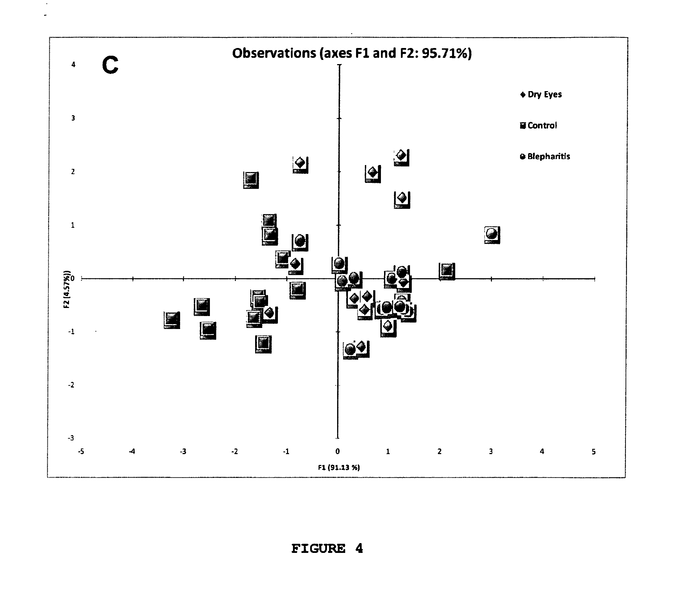

Figure 4 shows a graphical depiction of the discriminant analysis using Lipophilin A and Peroxiredoxin 5 (both isoforms). The samples from the control group are shown with a square (■), those of dry eyes with a rhombus (◆) and the samples from the blepharitis group with a circle (●).

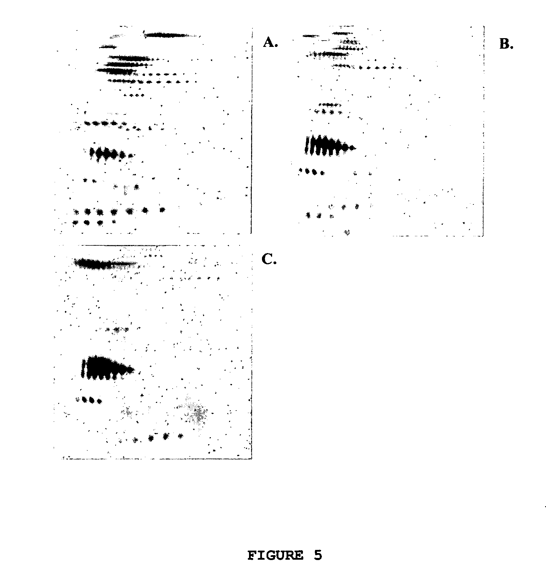

Figure 5 shows a tear proteome of a dry eyes (A), blepharitis (B) and control (C) patient. The 2-D electrophoresis has been performed in 17 cm strips with a pH range of 4-9 loading 40 µg of total tear protein of each patient, after its treatment with a Sigma Seppro column and an isoelectric focusing of 60623 Vxh. The second dimension has been performed in 15% polyacrylamide gels, which allows a separation between 10 and 250 kDa. The fluorescent reagent Rubipy was used for the viewing and the VersaDoc system was used as the as the fluorescence detection system.

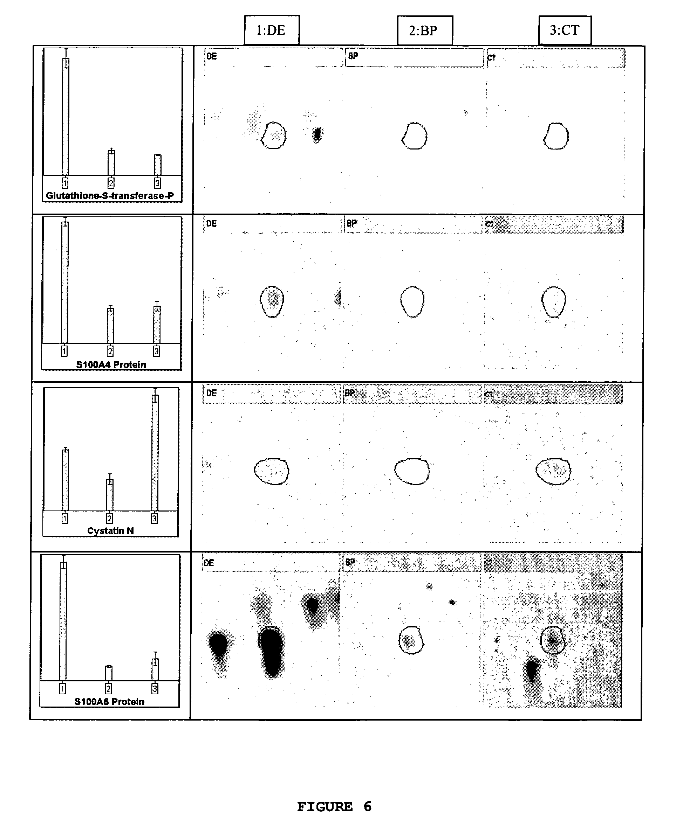

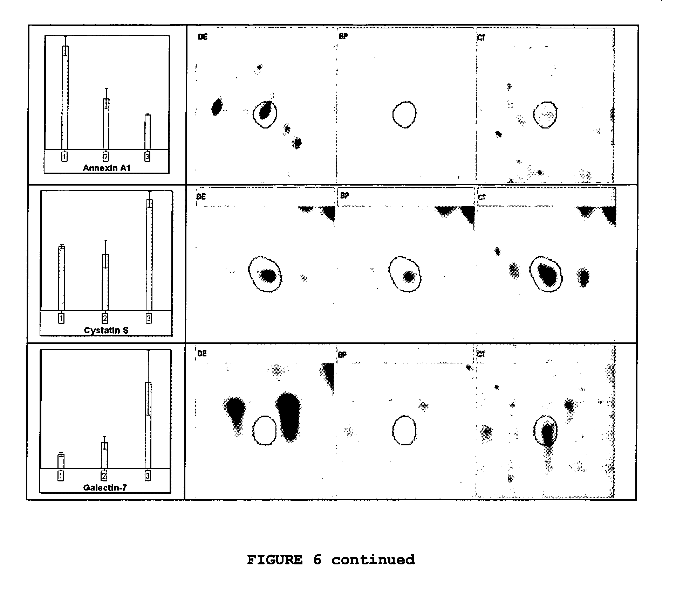

Figure 6 shows a proteome with the differential expression of the spots with the highest statistically significant variance of the study. At the left of each of the spots, the mean expression levels for each of the groups are depicted in the form of a bar graph, 1 being the dry eyes group, 2 the blepharitis group and 3 the control group, together with the name of the protein identified by means of MALDI-TOF/TOF mass spectrometry.

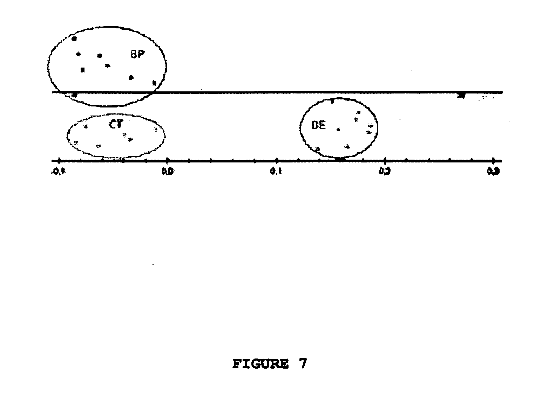

Figure 7 shows a depiction of the analysis of principal components using only those spots which are statistically significant in the comparison between the groups, i.e., 54 spots within the proteome with a statistical power greater than 0.9. The proteins identified as Group 1 in Table 5 are included within these spots. Each spot in the graph represents a gel within the study included in one of the following groups: DE: dry eyes; BP: blepharitis; CT: control. The x-axis shows the principal component 1 (PC1), which represents 73% of the variance, whereas the y-axis shows the principal component 2 (PC2) with 17.5% of the variance. Therefore, 90.5% of the variance between the groups of the study is explained between the first two principal components (PC1 and PC2). A perfect separation between the different groups can be observed, which indicates that the significantly altered proteins can be good diagnostic candidates to be able to perform a differentiation between the groups.

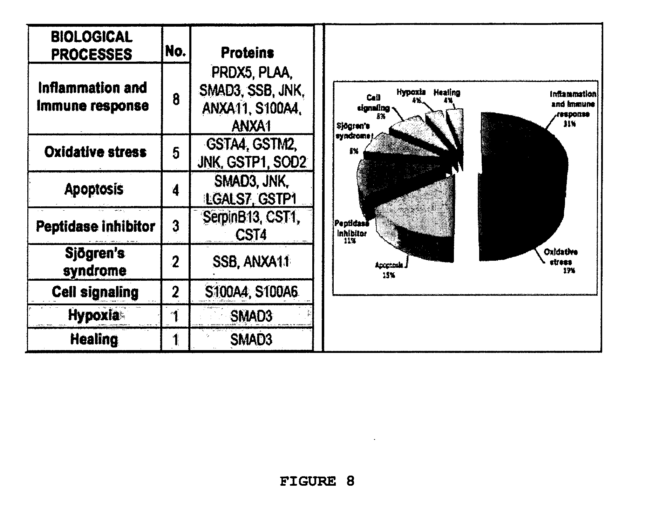

Figure 8 shows a diagram of the markers of the invention and their involvement in different biological processes. The table on the left shows the main biological processes in which each of the biomarker candidates obtained in this study are involved, together with their abundance. The percentages of frequency of the most abundant biological processes are schematized on the right by means of a circular depiction of proportions.

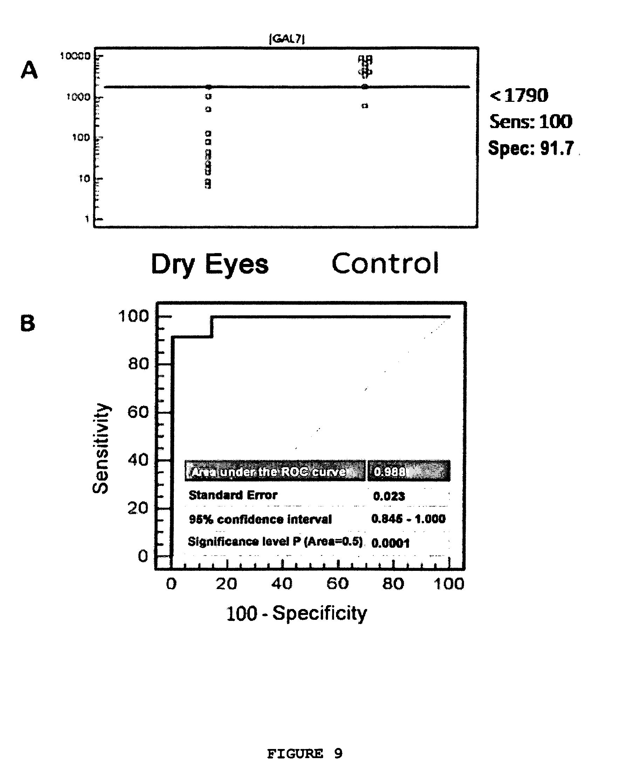

Figure 9 shows a dot plot at a logarithmic scale (A) in which the Galectin 7 concentration values obtained for the control group and for the pathological dry eyes group are compared. The critical point of Galectin 7 concentration is 1,790 pg/ml, which indicates that below said value it has 91.7% specificity and 100% sensitivity. (B) ROC curve for the Galectin 7 concentration values obtained from the ELISA assays. An area under the curve of 0.988 is verified, indicating that said marker could behave like an excellent diagnostic tool for the separation between the control and dry eyes patient groups.

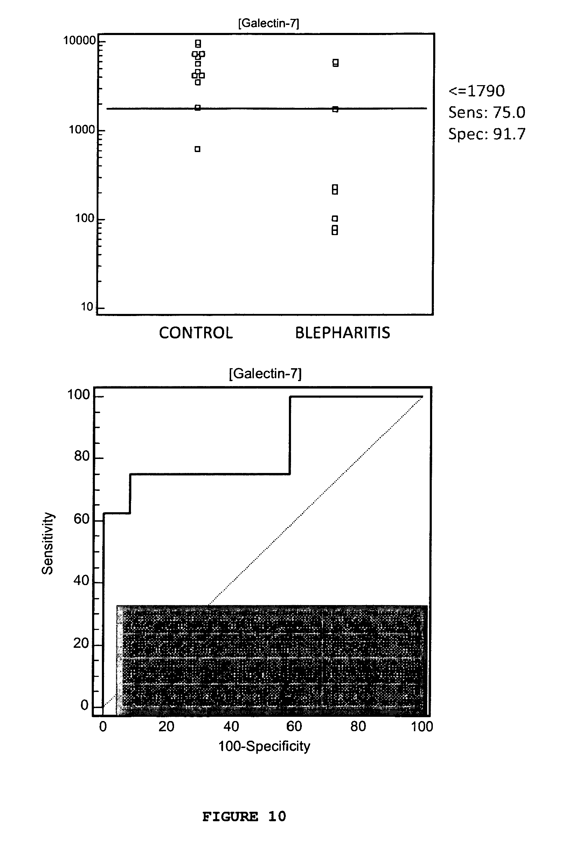

Figure 10 shows a dot plot at a logarithmic scale (A) in which the Galectin 7 concentration values obtained for the control group and for the pathological blepharitis group are compared. The critical point of Galectin 7 concentration is 1,790 pg/ml, which indicates that below said value it has 75% sensitivity and 91.7% specificity. (B) ROC curve for the Galectin 7 concentration values obtained from the ELISA assays. An area under the curve of 0.844 is verified, indicating that said marker could behave like an excellent diagnostic tool for the separation between the control and blepharitis patient groups.

### Detailed Description of the Invention

The inventors of the present invention have discovered a new group of molecular markers which allow the detection and/or the diagnosis of dry eye and blepharitis and, furthermore, allow discriminating between patients suffering from dry eye and blepharitis.

In a first aspect, the invention relates to an *in vitro* method for diagnosing dry eyes, hereinafter first method of the invention, in a subject which comprises:
(i) determining the level of one or more proteins selected from the group of Lipophilin A, Peroxiredoxin 5, Cystatin S, Cystatin N, S100A4, S100A6, GST P1, Annexin 1 and Galectin 7 or a functionally equivalent variant thereof, in a sample from a subject; and
(ii) comparing the level of said protein or proteins with the level of said protein or proteins in a reference sample,
   wherein if the subject has dry eye, the level of at least one of the S100A4, S100A6, GST P1 and Annexin 1 proteins is elevated with respect to its level in the reference sample and/or the level of at least one of the Cystatin S, Cystatin N, Galectin 7, Lipophilin A and Peroxiredoxin 5 proteins is reduced with respect to its level the reference sample.

In a particular embodiment, the first method of the invention additionally comprises determining the level of one or more proteins selected from the group of Annexin 11, Serpin B13, Reticulocalbin 1, Superoxide dismutase, SMAD3, JNK, PLAA, GSTM2, GSTA4 and SSB or a functionally equivalent variant thereof.

In another aspect, the invention relates to an *in vitro* method for diagnosing blepharitis, hereinafter second method of the invention, in a subject which comprises:
(i) determining the level of one or more proteins selected from the group of Lipophilin A, Peroxiredoxin 5, Cystatin S, Cystatin N, S100A4, S100A6, GST P1, Annexin 1 and Galectin 7 or a functionally equivalent variant thereof, in a sample from a subject; and
(ii) comparing the level of said protein or proteins with the level of said protein or proteins in a reference sample,
   wherein if the subject has blepharitis, the level of at least one of the GST P1 and Annexin 1 proteins is elevated with respect to its level in the reference sample, the level of the S100A4 protein remains identical with respect to its level in the reference sample and/or the level of at least one of the Cystatin S, Cystatin N, S100A6, Galectin 7, Lipophilin A and Peroxiredoxin 5 proteins is reduced with respect to its level in the reference sample.

In a particular embodiment, the second method of the invention additionally comprises determining the level of one or more proteins selected from the group of Annexin 11, Serpin B13, Reticulocalbin 1, Superoxide dismutase, SMAD3, JNK, PLAA, GSTM2, GSTA4 and SSB or a functional variant thereof.

In another aspect, the invention relates to an *in vitro* method for differentiating between dry eye and blepharitis, hereinafter third method of the invention, in a subject which comprises:
(i) determining the level of one or more proteins selected from the group of Lipophilin A, Peroxiredoxin 5, Cystatin S, Cystatin N, S100A4, S100A6, GST P1, Annexin 1 and Galectin 7 or a functionally equivalent variant thereof, in a sample from a subject; and
(ii) comparing the level of said protein or proteins with the level of said protein or proteins in a reference sample,
   wherein if the subject has dry eye, the level of at least one of the GSTP1, S100A6 and S100A4 proteins is elevated with respect to its level in the blepharitis sample and in the reference sample.

In a particular embodiment, the third method of the invention additionally comprises determining the level of one or more proteins selected from the group of Annexin 11, Serpin B13, Reticulocalbin 1, Superoxide dismutase, SMAD3, JNK, PLAA, GSTM2, GSTA4 and SSB.

In a particular embodiment, the sample from which the level of proteins is determined for the diagnosis of the pathologies, in the three methods of the invention, is a tear sample from a subject.

In another aspect, the invention relates to an *in vitro* method for diagnosing blepharitis or dry eye in a tear sample, hereinafter fourth method of the invention, from a subject which comprises:
(i) determining the level of Galectin 7 or a functionally equivalent variant thereof in said sample; and
(ii) comparing the level of said protein with the level of the protein in a reference sample,
   wherein if the subject has dry eye, the level of Galectin 7 is reduced with respect to its level in a reference sample.

The Galectin 7 protein can individually serve as a marker for the diagnosis of blepharitis or dry eye in a tear sample from a subject. In both cases, in subjects with blepharitis and in subjects with dry eyes, it is observed that the level of the Galectin 7 protein is reduced with respect to the level of Galectin 7 in a reference sample. Therefore, the fourth method of the invention is based on the determination of the level of said protein in a tear sample and said level is compared with the level of said protein in a reference sample, allowing the differentiation of subjects with blepharitis or dry eyes from healthy subjects, wherein if the level of Galectin 7 is reduced with respect to the level of said protein in a reference sample, it indicates that the subject has blepharitis or dry eyes. For the determination of the level of the Galectin 7 protein and the details of the method, reference is made to the methods described for the rest of the methods of the invention throughout the specification.

The first three methods of the invention, the first, second and third method of the invention, are based on the detection and quantification of the proteins in an individual manner or combinations of said proteins, i.e., one, two, three, four, five, six, seven, eight or nine of the aforementioned proteins or combinations of two, of three, of four, of five, of six, of seven or of eight proteins and even the nine proteins. Likewise, the determination of the level of other proteins (Annexin 11, Serpin B13, Reticulocalbin 1, Superoxide dismutase, SMAD3, JNK, PLAA, GSTM2, GSTA4 and SSB), also individually or combined, can additionally be included in said methods of the invention.

As used in the present invention, the term "subject" relates to any mammalian animal and includes but is not restricted to domestic animals, farm animals, primates and humans, such as human beings, non-human primates, cows, horses, pigs, sheep, dogs, cats or rodents.

The term "marker" will be used indistinctly throughout the invention together with the term "protein".

As used herein, the term "sample" relates to any biological sample which can be obtained from the eye of a subject, such as conjunctiva, tears, cornea, aqueous humor, etc. Preferably, for putting the first three methods of the invention into practice, said sample is a tear sample from the subject. The tear sample can be obtained by conventional methods known by the person skilled in the art, for example, by means of an absorbent paper, a capillary or an eye spear. The sample can be obtained from subjects previously diagnosed, or not diagnosed with said pathologies, or also from a subject undergoing treatment, or who has been previously treated for one of said pathologies.

The present invention relates to methods for diagnosis comprising the determination of the level of one or more proteins in a sample from a subject. Said proteins correspond to the following:

### Lipophilin A

This protein, also referred to as "Secretoglobin family 1D member 1" (SG1D1), the accession number of which in the UniProt database on 27 July 2010 is P30044 (SEQ ID NO: 18), is found forming a heterodimer with Lipophilin C. It is expressed in lacrimal glands, thymus, kidneys, testicles, ovaries and salivary glands. It is known that it can bind to androgens and other steroids and to estramustine, in addition to being a chemotherapeutic agent used in prostate cancer. It can also be found under transcriptional regulation of steroid hormones.

### Peroxiredoxin 5

The Peroxiredoxin 5 (PRDX5) protein reduces hydrogen peroxide and alkyl hydroperoxides and is involved in intracellular redox signaling, in inflammatory response and in cell response to reactive oxygen species. Its accession number in the UniProt database on 27 July 2010 is P30044 (SEQ ID NO: 19).

### Cystatin S

Also referred to as Cystatin 4 and CST4, it is a protein which is secreted and strongly inhibits papain and ficin, as well as partially stem bromelain and bovine cathepsin C. It is found in saliva, tears, urine and seminal fluid. Its accession number in the UniProt database on 27 July 2010 is P01036 (SEQ ID NO: 1).

### Cystatin N

This protein, also called Cystatin SN, Cystatin 1 and CST1, the accession number of which in the UniProt database on 27 July 2010 is P01037 (SEQ ID NO: 2), is found in saliva, tears, urine and seminal fluid. It is a very strong inhibitor of papain and of dipeptidyl peptidase I and to a lesser extent of ficin.

### S100A6

It is a calcium-binding protein, the accession number of which in the UniProt database on 27 July 2010 is P06703 (SEQ ID NO: 3). It can interact with other proteins and has an indirect role in the reorganization of the actin cytoskeleton and in cell motility. Annexin 11 is among the proteins with which it interacts.

### S100A4

It is a calcium-binding protein, the accession number of which in the UniProt database on 27 July 2010 is P06702 (SEQ ID NO: 4). It is located in the cytoplasm and/or nucleus of many cell types and is involved in the regulation of the cell cycle progression and in cell differentiation. Specifically, this protein can participate in motility, invasion and polymerization of tubulin, whereas its deficiency has been related to tumor metastasis.

### Glutathione S-transferase P

It is also called GST P1. It is a homodimer, the accession number of which in the UniProt database on 27 July 2010 is P09211 (SEQ ID NO: 5). Its function is the conjugation of reduced glutathione to a large number of both exogenous and endogenous hydrophobic electrophiles.

### Annexin 1

Annexin 1 (ANX a1) is a calcium/phospholipid-binding protein promoting the fusion of membranes and is involved in exocytosis. This protein, the accession number of which in the UniProt database on 27 July 2010 is P04083 (SEQ ID NO: 7), regulates the activity of phospholipase A2 and is believed to bind between 2 and 4 calcium ions with high affinity.

### Galectin 7

It is believed that Galectin 7 (LGALS7) may be involved in cell-cell and/or cell/matrix interactions, necessary for the control of cell growth. This protein, the accession number of which in the UniProt database on 27 July 2010 is P47929 (SEQ ID NO: 6), is induced by p53.

### Annexin 11

Annexin 11 (ANX a11) binds specifically to calcyclin, in a calcium-dependent manner. This protein, the accession number of which in the UniProt database on 27 July 2010 is P50995 (SEQ ID NO: 17), interacts with the S100A6 protein. Anti-ANXa11 antibodies have been found in the serum of patients with some autoimmune diseases, such as rheumatoid arthritis, systemic lupus erythematosus or Sjögren's syndrome.

### Serpin B13

It is believed that the Serpin B13 protein may be involved in keratinocyte proliferation or differentiation. This protein, the accession number of which in the UniProt database on 27 July 2010 is Q9UIV8 (SEQ ID NO: 8), is specifically expressed in the skin.

### Reticulocalbin 1

This protein, also referred to as RCN1 and the accession number of which in the UniProt database on 27 July 2010 is Q15293 (SEQ ID NO: 9), has four binding sites for calcium, although only two of them are active. It is capable of regulating the calcium-dependent activities in the lumen of the endoplasmic reticulum or post-endoplasmic reticulum compartment.

### Superoxide dismutase

Superoxide dismutase (SOD2) destroys free radicals which are normally produced inside the cells and which are toxic for said cells. Genetic variations of SOD2 (the accession number of which in the UniProt database on 27 July 2010 is P04179 (SEQ ID NO: 10)) have been associated with the susceptibility of suffering from diabetic nephropathy.

### SMAD3

It is thus referred to because of its name "Mothers against decapentaplegic homolog 3". Its accession number in the UniProt database on 27 July 2010 is P84022 (SEQ ID NO: 11). It is a transcriptional modulator activated by TGF-beta and by Activin Receptor-Like Kinases Type I. Defects in said protein may be the cause of colorectal cancer.

### JNK

It is also referred to as "Stress-activated protein kinase" or MAPK8 and the accession number of which in the UniProt database on 27 July 2010 is P45983 (SEQ ID NO: 12). It responds to activation by environmental stress and pro-inflammatory cytokines, phosphorylating a large number of transcription factors, first of all AP-1 components, such as JUN, JDP2 and ATF2 and it then regulates the transcriptional activity of AP-1. It has three isoforms: 1, 2, and 3 and each one has different binding patterns, although the phosphorylation efficiency coincides in all the isoforms.

### PLAA

It is also called phospholipase A2 activator protein" and the accession number of which in the UniProt database on 27 July 2010 is Q9Y263 (SEQ ID NO: 13), it has an important role in the regulation of specific inflammatory pathological processes, regulating the activity of phospholipase A2.

### GSTM2

The GSTM2 protein is also referred to as "Gluthatione S-transferase Mu2" and its accession number in the UniProt database on 27 July 2010 is P28161 (SEQ ID NO: 14). It is capable of conjugating reduced glutathione to a large number of exogenous and endogenous hydrophobic electrophiles.

### GSTA4

The GSTA4 protein is also referred to as "Glutathione S-transferase A4" and its accession number in the UniProt database on 27 July 2010 is 015217 (SEQ ID NO: 15). Like the GSTM2 protein, it is capable of conjugating reduced glutathione to a large number of exogenous and endogenous hydrophobic electrophiles.

### SSB

The SSB protein, also called "Lupus La protein" or "SS type B antigen" has an important role in the transcription of RNA polymerase III. It is believed that this protein, the accession number of which in the UniProt database on 27 July 2010 is P05455 (SEQ ID NO: 16), is a transcription termination factor. It acts by binding to the 3' end of the nascent transcripts of RNA polymerase III. It is involved in the metabolic process of the mRNA of histones and in the modification of tRNA.

The aforementioned proteins are identified in a schematized form in the following table, together with their accession number in the Uniprot database on 28 July 2010 and the sequence identifier of the sequence listing (SEQ ID NO).

| **Gene** | **Protein** | **UniProt/ SwissProt** | **SEQ ID NO:** |
|---|---|---|---|
| **CST4** | Cystatin S = Cystatin 4 | P01036 | 1 |
| **CST1** | Cystatin N = Cystatin 1 | P01037 | 2 |
| **S100A6** | S100 calcium-binding protein A6 | P06703 | 3 |
| **S100A4** | S100 calcium-binding protein A4 | P06702 | 4 |
| **GSTP1** | Glutathione S-transferase P | P09211 | 5 |
| **LGALS7** | Galectin 7 | P47929 | 6 |
| **ANX A1** | Annexin 1 | P04083 | 7 |
| **Serpin B13** | Serpin B13 | Q9UIV8 | 8 |
| **RCN 1** | Reticulocalbin 1 | Q15293 | 9 |
| **SOD2** | Superoxide dismutase | P04179 | 10 |
| **SMAD3** | Mothers against decapentaplegic homolog 3 | P84022 | 11 |
| **JNK** | Stress-activated protein kinase = MAPK8 | P45983 | 12 |
| **PLAA** | Phosp. A2 activator protein | Q9Y263 | 13 |
| **GSTM 2** | Glutathione S-transferase Mu 2 | P28161 | 14 |
| **GSTA 4** | Glutathione S-transferase A4 | 015217 | 15 |
| **SSB** | Lupus La protein, SS type B antigen | P05455 | 16 |
| **ANX A11** | Annexin 11 | P50995 | 17 |
| **SG1D1** | Secretoglobin family 1D member 1 (Lipophilin A) | 095968 | 18 |
| **PRDX5** | Peroxiredoxin 5 | P30044 | 19 |

A skilled person in the art will understand that the present invention relates not only to the specific proteins mentioned in the present invention, but also to variants thereof. Therefore, the term "functionally equivalent variant" of the proteins of the invention, according to the present invention, can relate to (i) a variant in which one or more amino acids are substituted with a conserved or non-conserved amino acid; (ii) a variant in which there are one or more modified amino acids, such as residues modified by the binding of substituent groups; (iii) variants in which the protein is a processing or splicing alternative and/or (iv) fragments of the protein. The fragments include proteins generated by proteolytic cleavage. As is known in the state of the art, the identity between two proteins is determined by comparing the amino acid sequence of a first and a second protein. Variants according to the present invention include amino acid sequences having at least 60%, 65%, 70%, 72%, 74%, 76%, 78%, 80%, 90%, or 95% similarity or identity with the original amino acid sequence. The degree of identity between two proteins is determined by using computational algorithms and methods which are widely known by the persons skilled in the art. The identity between two amino acid sequences will preferably be determined by using the BLASTP algorithm [BLASTManual, Altschul, S., et al., NCBI NLM NIH Bethesda, Md. 20894, Altschul, S., et al., J. Mol. Biol. 215: 403-410 (1990)]. Said functionally equivalent variants will conserve the capacity to be used as markers for the diagnosis of dry eye or blepharitis, as shown in the Examples of the present invention. Likewise, the variants according to the invention can include post-translational modifications, such as glycosylation, acetylation, isoprenylation, myristoylation, proteolytic processing, etc.

In the context of the present invention, the level of a protein is said to be increased when the level of said protein is elevated with respect to the levels of said protein in the control or reference sample. According to the present invention, the levels of protein are considered to be increased with respect to the levels of said proteins in the reference sample when the levels of protein in the sample from the subject are increased by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%: at least 85%, at least 90%, at least 95%, at least 100%, at least 110%, at least 120%, at least 130%, at least 140%, at least 150% or more.

On the other hand, in the context of the present invention, the level of a protein is said to be reduced or repressed when the level of said protein is reduced with respect to the levels of the protein in the control or reference sample. According to the present invention, the levels of protein are considered to be reduced with respect to the levels of said protein in the reference sample when the levels of protein in the sample from the subject are reduced by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%: at least 85%, at least 90%, at least 95%, at least 100%, at least 110%, at least 120%, at least 130%, at least 140%, at least 150% or more.

Table 1 indicates the level of the proteins/markers which are analyzed in the first method of the invention, i.e., if the level of the protein is elevated (+) with respect to the level of said protein in the reference sample or reduced or repressed (-) with respect to the level of said protein in the reference sample. On the other hand, Table 2 indicates the level of the proteins/markers which are analyzed in the second method of the invention, i.e., if the level of the protein is elevated (+) with respect to the level of said protein in the reference sample or reduced, repressed (-) with respect to the level of said protein in the reference sample, or if the level of the protein is unchanged (=) with respect to the level of said protein in the reference sample. Table 1 and Table 2 also include the number of times (fold) by which the level of the protein is altered with respect to the level of said protein in the reference sample.

| **Gene** | **Protein** | **Dry eye** | **Fold** |
|---|---|---|---|
| **CST4** | Cystatin S = Cystatin 4 | - | - 1.78 |
| **CST1** | Cystatin N = Cystatin 1 | - | - 1.89 |
| **S100A6** | S100 calcium-binding protein A6 | + | 5.39 |
| **S100A4** | S100 calcium-binding protein A4 | + | 3.28 |
| **GST P1** | Glutathione S-transferase P | + | 5.71 |
| **LGALS7** | Galectin 7 | - | - 6.21 |
| **ANX A1** | Annexin 1 | + | 2.96 |
| **SG1D1** | Lipophilin A (Secretoglobin family 1D member 1) | - | - 2.00 |
| **PRDX5** | Peroxiredoxin 5 | - | - 1.14 |

Table 1: Levels of each of the proteins detected in the first method of the invention. The (-) sign indicates that level of said protein is reduced or repressed with respect to the level of the protein in the reference sample, whereas the (+) sign indicates that the level of said protein is increased with respect to the level of the protein in the reference sample.

| **Gene** | **Protein** | **Blepharitis** | **Fold** |
|---|---|---|---|
| **CST4** | Cystatin S = Cystatin 4 | - | - 1.96 |
| **CST1** | Cystatin N = Cystatin 1 | - | - 3.64 |
| **S100A6** | S100 calcium-binding protein A6 | - | - 1.53 |
| **S100A4** | S100 calcium-binding protein A4 | - | - |
| **T P1** | Glutathione S-transferase P | + | 1.20 |
| **LGALS7** | Galectin 7 | - | - 3.37 |
| **ANX A1** | Annexin 1 | + | 1.45 |
| **SG1D1** | Lipophilin A (Secretoglobin family 1D member 1) | - | - 2.12 |
| **PRDX5** | Peroxiredoxin 5 | - | - 1.62 |

Table 2: Levels of each of the proteins detected in the method of the invention for blepharitis. The (-) sign indicates that the level of said protein is reduced or repressed with respect to the level of the protein in the reference sample, whereas the (+) sign indicates that the level of said protein is increased with respect to the level of the protein in the reference sample. The (=) sign indicates that the level of said protein is at the same level with respect to the level of the protein in the reference sample.

In the context of the present invention, the term "reference sample" or control sample is understood as the biological sample from a healthy subject which is used to determine the variation of the levels of the proteins used in the present invention. In an embodiment, the level of the protein in the reference sample is obtained from the signal provided by using a sample from a healthy individual, who has neither dry eye syndrome nor blepharitis, nor has undergone processes of eye surgery, allergy, atopy, nor medication with corticoids or antiglaucoma agents.

The determination of the levels of the proteins or markers can be carried out by means of immunological techniques, such as ELISA (Enzyme-Linked Immunosorbent Assay, Western blot, RIA (radioimmunoassay), competitive EIA (competitive enzyme immunoassay), DAS-ELISA (Double Antibody Sandwich ELISA), immunocytochemical and immunohistochemical techniques, techniques based on the use of protein biochips or microarrays including specific antibodies or assays based on colloidal precipitation in formats such as dipsticks. The Western blot technique is based on the detection of proteins previously separated by electrophoresis in a gel, under denaturing conditions and immobilized in a membrane which is subsequently incubated with one or more antibodies specific for the protein and is detected by means of a system, for example, a chemiluminescent or fluorescent system. The immunofluorescence analysis requires the use of a specific antibody labeled with a fluorescent compound. The ELISA technique is based on the use of antigens or antibodies labeled with enzymes, such that the conjugates formed between the target antigen and the labeled antibody results in the formation of enzymatically active complexes. Due to the fact that one of the components (the antigen or the labeled antibody) is immobilized in a support, the antibody-antigen complexes are also immobilized in the support and therefore they can be detected by adding a substrate which is converted by the enzyme into a product which is detectable by spectrophotometry or fluorometry, for example. The proteins are preferably detected by Western blot, ELISA, a protein array or by two-dimensional electrophoresis.

The skilled person in the art will understand that, if the level of the proteins is to be determined by two-dimensional electrophoresis, the gel must be stained for the purpose of detecting said proteins and it must be done such that it is quantitative. Any staining method sensitive enough to detect the proteins/markers of the invention can be used and includes but is not limited to silver staining, Coomassie blue staining, Sypro Ruby staining, fluorescent cyanine staining, such as that described in Mujumdar, R. B. et al., (Cytometry, 1989, 10:11-19 and US5268486), dipyrrometheneboron difluoride staining, such as that described in US4774339, etc. Once the spots showing statistically significant differences between the reference sample and the sample from the patient are identified, the proteins described in the method of the invention are identified according to their molecular weight and their isoelectric point. The identity of the protein spots identified in the two-dimensional gel can be confirmed by cutting the spot from the gel, carrying out an in-gel digestion and subsequent mass spectrometry. The levels of protein in the selected spots can be quantified by using any known technique, such as stained gel densitometry and determination of the volume (intensity x height), differential in-gel electrophoresis (DIGE), wherein the proteins to be separated are labeled with fluorophores as a step prior to electrophoretic fractionation and they are then mixed with an internal standard, representing all the proteins in the sample and which can be used for normalization purposes. Likewise, the regions of the gels in which the proteins are differentially expressed can be cut and the identity of the spot of the protein can be identified by using techniques such as MALDI (MALDI-MS/MS) mass spectrometry, electrospray ionization mass spectrometry (ESI-MS/MS). Alternatively, peptide digestion can be carried out and the peptide profile can be determined by mass spectrometry, Edman degradation sequencing or n N-terminal sequencing.

The final step of the four methods of the invention comprises comparing the level of the protein or proteins with the level or the amount of said protein-marker detected in the reference samples from control subjects or in previous samples from the same individual.

Additionally, if desired, the determination of the level of proteins in a sample from an individual having one of the pathologies can be incorporated to the four methods of the invention as a positive control, such that it is verified that the method of the invention is effective.

In another aspect, the invention relates to a kit useful in the implementation of the methodology described in the present invention. Thus, said kit comprises at least one reagent for the detection of at least one protein selected from the group of Lipophilin A, Peroxiredoxin 5, Cystatin S, Cystatin N, S100A4, S100A6, GST P1, Annexin 1 and Galectin 7 or a functional variant thereof. In other words, the kit includes a set of reagents for detecting the expression levels of all the proteins or of at least one, two, three, four, five, six, seven, eight or nine of the proteins, or functionally equivalent variants of said proteins. Thus, the kit comprises the reagents for the detection of one or more of the following proteins: Lipophilin A, Peroxiredoxin 5, Cystatin S, Cystatin N, S100A4, S100A6, GST P1, Annexin 1 and Galectin 7 or functionally equivalent variants thereof.

In a particular embodiment, the invention relates to a kit comprising the aforementioned reagents and, additionally, reagents for the detection of one or more proteins selected from the group of Annexin 11, Serpin B13, Reticulocalbin 1, Superoxide dismutase, SMAD3, JNK, PLAA, GSTM2, GSTA4 and SSB.

The kit of the invention optionally comprises a positive control including a known sample, which has a pattern of levels of proteins which coincides with a sample from a subject having dry eye or blepharitis.

In another aspect, the kit of the invention is used in the *in vitro* diagnosis of dry eye or blepharitis or for differentiating between both pathologies.

The invention is illustrated below based on the following examples which are provided to illustrate but not to limit the scope of the invention.

### EXAMPLES

### MATERIALS AND METHODS

### Tear sampling:

Tear samples were taken from different subjects with an eye spear (PVA 0525; Oasis, Glendora, CA), without applying any type of anesthesia. The eye spears were then left in an Eppendorf tube containing 200 µl of PBS (phosphate buffer) for 1 hour for the purpose of achieving a more efficient elution of the proteins. The protein material was collected by means of centrifugation at 4°C and stored at -80°C until the time of the analysis. The samples were classified according to the inclusion and exclusion criteria mentioned below:
Exclusion Criteria:
   - Eye surgery (2 months ago)
   - History of allergies
   - Atopy
   - Medications with corticoids
   - Chronic medications (Glaucoma)
   - Any medication except artificial tears
   - Contact lenses

General Inclusion Criteria:
- Age above 18 years
- Signing the informed consent

Inclusion Criteria by group:
- Healthy controls:
   a. Absence of dry eyes and/or blepharitis symptoms
   b. Jones test >5 mm
   c. Not contact lenses wearers
   d. No atopy
- Dry eyes:
   a. Dryness or burning sensation symptoms which worsen in dry environments and throughout the day
   b. Jones test <5 mm
- Blepharitis:
   a. Eyelid inflammation
   b. Eye discomfort and episodic reddening symptoms
   c. Vascularization in eyelid edge
   d. Itching
   e. Jones test >5 mm
   f. Slit lamp: hyperemia and thickening of the free eyelid edge; meibomian gland dysfunction; instability of the tear film (with fluorescein).

### EXAMPLE 1: Validation of markers by Western blot

### MATERIALS AND METHODS

### 1D-SDS-PAGE and Western Blot:

Samples were taken from 39 subjects in order to carry out the process for validating markers previously described in the literature (Lipophilin A and Peroxiredoxin 5). Thus, according to the aforementioned inclusion and exclusion criteria, the 39 samples from subjects were divided as follows: 13 individuals of the control group, 12 individuals belonging to the group of patients with dry eyes and 14 individuals belonging to the group of patients with blepharitis. The sample was collected in exactly the same way as in the case of the tear samples obtained in Example 2.

For the validation of the Lipophilin A and Peroxiredoxin 5 markers, a comparative expression study was carried out by means of SDS-PAGE electrophoresis in 15% acrylamide gels followed by immunofluorescent multiplex western blot on low-fluorescence PVDF (Polyvinylidene Fluoride) membranes. To carry out this assay, for the detection of Peroxiredoxin (PRDX5), a primary anti-PRDX5 antibody produced in mice was used and a secondary anti-mouse antibody conjugated with AlexaFluor 633 nm (Invitrogen Dynal AS, Oslo, Norway) was used for its detection. The detection of lipophilin A was performed using goat anti-SG1D1 and a secondary anti-goat antibody conjugated with AlexaFluor 488 nm (Invitrogen Dynal AS, Oslo, Norway) for its detection. The idea of performing this multiplex assay consists of being able to use the same samples for analyzing several markers using two different excitation/emission channels, and leaving a third one for the normalization by means of total protein staining with Sypro Ruby® (Invitrogen Dynal AS, Oslo, Norway). The optimal conditions of antibodies for the detection of the proteins were established, using a 1:1000 ratio for the primary antibodies and a 1:5000 ratio for the secondary antibodies.

The fluorescence volume value (area x fluorescence intensity) of the band corresponding to each of the markers was extracted from each of the Western blots and normalized with respect to the total protein value. The corresponding statistical analyses (ANOVA, Student's t-test, discriminant analysis, dot-plot interactions and linear regression) were subsequently performed.

### RESULTS

In the Western blots, in the case of Peroxiredoxin 5, the appearance of two isoforms, which were referred to as A and B (A being the one with the highest molecular mass), was detected, so each of them were assessed individually and, also, both isoforms were assessed jointly. Figure 1 shows the results of the Western blot for Lipophilin A and for Peroxiredoxin 5, for dry eyes and blepharitis, compared with the controls. Figure 2 depicts the normalized volume values for each group and for each marker. A reduction in the Lipophilin A concentration, both in dry eyes and in blepharitis with respect to the control, can be observed. In the case of Peroxiredoxin 5, both in dry eyes and in blepharitis, the presence of an isoform (PRDX5-A) which was virtually not present in the control group is observed.

After performing the corresponding ANOVA and Student's t-test statistical analyses, it was possible to verify that both Lipophilin A and Peroxiredoxin 5 were capable of differentiating each of the pathologies with respect to the control with probability values (p-values) lower than 0.05. Lipophilin A presented a statistically significant difference between the control group and the blepharitis group. Isoform A of Peroxiredoxin 5 was capable of differentiating the control group both from dry eyes and from blepharitis, whereas isoform B of Peroxiredoxin 5 was capable of exclusively differentiating between blepharitis and controls, indicating its potential as a differential diagnostic marker for blepharitis. Finally, taking into account both isoforms together, a differentiation of dry eyes versus the blepharitis group was observed, which is a very important piece of data since it is a differential diagnosis between both pathologies with respect to the control. Table 3 shows the probability values associated with these analyses, highlighting in gray those which are significant with a confidence level of 95% (p<0.05).

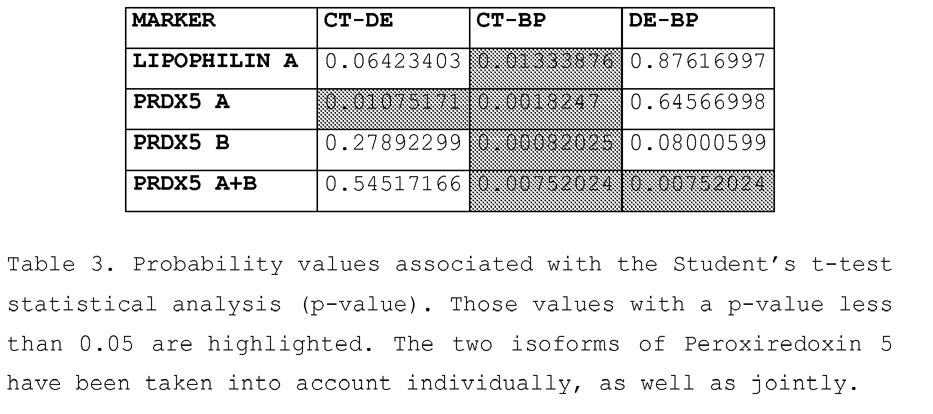

Likewise, when the data were analyzed by means of simple linear regression using logical values for the independent variable (0 and 1), the result obtained allows determining the prediction capacity which is obtained from the data. In other words, if the relationship between the groups is linear, slope b associated with the straight line estimates the effect" of the independent variable on the dependent variable, and allows analyzing the hypothesis of independence between the two variables. Furthermore, the intensity of the relationship is evaluated with the R² index and predictions can be made from the model. When the logistic regression which is performed is simple it is verified that, despite the analyzed markers being capable of differentiating between the different groups, the percentage of patients the group of which could be predicted in the event of using a single marker would be 85.19% for the comparison of the blepharitis pathology with the control group. However, when the regression analysis is approached taking into account the 2 variables present in the system (Peroxiredoxin 5 and Lipophilin A values), the effectiveness in the prediction increases up to 92.59%, a value which indicates the percentage of correctly classified cases.

The correct classification of the cases has also been approached by means of studying ROC curves and interactive dot plots, as shown in Figure 3, which depicts the normalized volume values obtained for Peroxiredoxin 5 by means of Western blot. It can be observed that the sensitivity and specificity values in both dry eyes-control and blepharitis-control comparisons are suitable, indicating that they can be suitable markers for both pathologies.

In addition, upon performing discriminant analysis as a multivariate statistical tool and using values of both Lipophilin A and of Peroxiredoxin 5, isoforms A and B, for the analysis, a suitable clustering of all the classes was seen, as shown in Figure 4. Likewise, a perfect clustering of the dots of the control group was observed, except for a single dot. It can also be observed that the rest of the groups are well defined although overlapping one another with the exception of the dry eyes group, which forms two independent clusters separated from one another. The two subgroups found within dry eyes will probably be due to the type of the pathology or to the degree of progress of said pathology. However, despite the fact that the control group is well separated from the rest, this analysis also indicates an overlapping between the groups of patients with blepharitis and dry eyes. The classification of the individuals by means of learning techniques indicates that it is possible to differentiate between the three groups in 69.3% of the cases, with a correct classification maximum of 92.9% in the case of blepharitis.

Using this statistical methodology it was possible to determine the minimum number of markers necessary to predict to which group from among the three of the study (dry eyes, blepharitis and controls) any test sample belongs.

### EXAMPLE 2: Identification of markers by two-dimensional electrophoresis

### MATERIALS AND METHODS

According to the aforementioned inclusion and exclusion criteria, samples were collected from 44 subjects, which were divided as follows: 16 of them belonged to the group of patients with blepharitis, 16 to patients with dry eyes and 12 to control individuals.

In this case, the tear samples were processed to remove IgA and Seralbumin by means of immunoaffinity chromatography using a Seppro column (Sigma-Aldrich, St. Louis, MO, United States). Two of the major proteins of tears were thus removed from the samples, leaving other less abundant proteins more accessible, both for the analysis and for their subsequent identification. The flow-through sample fraction (not retained in the column) was precipitated with the CleanUp kit (GE Healthcare, Sunnyvale, CA, United States) for the removal of lipids and salts present in the samples following the protocol described by the manufacturer. After resuspending the pellets resulting from the precipitation in 100 µl of DeStreak (GE Healthcare, Sunnyvale, CA, United States), the samples were left solubilizing for 24 hours and they were quantified by means of a fluorometric technique based on the staining of total protein on paper (EZQ Invitrogen Dynal AS, Oslo, Norway).

One of the problems encountered when working with tears is the small amount of protein content obtained from each subject, due to the reduced volume obtained in the tear samples. The requirements in terms of the total protein load in order to be able to perform the 2D gels in the 17 cm format are a total of 40 µg per gel. In addition to the fact that most of the samples do not meet this requirement, a large part of this protein content is removed by means of immunoaffinity chromatography. As a result, some samples are obtained which do not reach the amount of protein required for the study and therefore these patients cannot be included in the study. For this reason, the samples of the same class were grouped into twos in an equimolar manner, and finally 8 gels of the blepharitis group (16 patients), 6 gels of the control group (12 individuals) and 8 gels of the dry eyes group (16 patients) were analyzed. The differential expression analysis of the resulting gels was carried out using the Progenesis SameSpots computer program (NonLinear Dynamics, North Carolina, USA).

40 µg of total protein of each of the pools of the samples were taken to a volume of 300 µl using DeStreak® (GE Healthcare, Sunnyvale, CA, United States) and supplementing with DTT (dithiothreitol) to a final concentration of 20 mM. All the samples were then focused in 17 cm strips with a pH range of 4-9, using the same number of Vxh and in a Protean IEF Cell device (BioRad, Malvern, Pennsylvania, USA), following a protocol which consisted of 2 stages: after 7 hours of passive hydration have elapsed, an active hydration was carried out at 50 V for 8 hours; the voltage was then taken to 10,000 V in an exponential gradient and the strips were left "focussing" for 60,000 Vxh in order to be used immediately afterwards in the second dimension. The already focused strips were equilibrated with equilibration buffer I (6 M Urea, 2% SDS, 29.3% glycerol, 2% Dithiothreitol (DTT)) for 15 minutes followed by another 15 minutes of equilibration with equilibration buffer II (6 M Urea, 2% SDS, 29.3% glycerol, 2.5% Iodoacetamide (IA)). Once reduced and carboxymethylated, the strips were resolved according to their molecular mass by means of SDS-PAGE electrophoresis under denaturing conditions in large 15% polyacrylamide gels (20x26 cm) using the DALT-SIX electrophoresis system (GE Healthcare, Sunnyvale, CA, United States) according to the standard protocol. The gels were viewed using a VersaDoc image analyzer (BioRad, Malvern, Pennsylvania, USA) after staining the gels with Sypro Ruby® (Invitrogen Dynal AS, Oslo, Norway) following the indications of the manufacturer.

### RESULTS

Figure 5 shows an image of the proteome of each of the study groups, in which the high level of resolution which was reached in the tear analysis (two-dimensional gels) can be observed.

The differential expression analysis of the resulting gels by means of the Progenesis SameSpots computer program shows that although there are variations in the expression levels throughout the entire gel, it can be emphasized that the most significant changes occur at low molecular mass. As a result, a series of spots differentially expressed between the different groups was found, as shown in Figure 6.

The multivariate statistical analysis (principal component analysis - PCA) indicated in Figure 7 performed from the raw data of expression of those spots which are statistically significant assured a perfect separation between the different groups. This perfect separation between groups indicates that the selected spots could be potential biomarkers since they are capable of differentiating the groups completely, therefore said spots have been identified by means of MALDI-TOF/TOF mass spectrometry. The results obtained, associated with the changes of expression and ANOVA values, are shown in Table 4.

| | | | **Normalized volume** | | |
|---|---|---|---|---|---|
| **Anova (p)** | **Fold** | **Protein** | **DE** | **BP** | **CT** |
| 9.74E-05 | 5.71 | Glutathione S-transferase P | 4.57E+05* | 9.61E+04 | 8.00E+04** |
| 6.03E-04 | 3.28 | S100-A4 Protein | 5.87E+05* | 1.71E+05** | 1.79E+05 |
| 0.001 | - 3.7 | Cystatin-N | 2.63E+05 | 1.36E+05** | 4.95E+05* |
| 0.002 | 5.39 | S100-A6 Protein (isoform 1) | 2.19E+06* | 2.66E+05** | 4.06E+05 |
| 0.007 | 3 | Annexin A1 | 2.26E+05* | 1.11E+05 | 7.11E+04* |
| 0.009 | 4.04 | S100-A6 Protein (isoform 2) | 8.05E+05* | 1.49E+05** | 1.99E+05 |
| 0.023 | - 1.96 | Cystatin-S | 1.29E+06 | 1.14E+06 | 2.23E+06 |
| 0.011 | - 6.2 | Galectin-7 | 5.54E+04 | 1.02E+05 | 3.44E+05 |

| | | | | | |
|---|---|---|---|---|---|
| [The values indicated with * correspond to the highest values, and the values indicated with ** to the lowest values. | | | | | |

Table 4: Results obtained associated with changes of expression and ANOVA values of several markers with respect to their different expression in the different groups of patients (DE) dry eyes, (BP) blepharitis and (CT) control group. The normalized volume values of the proteins with greater variation between the groups and statistically significant values are shown. The Fold" value (or number of times) has been calculated taking the highest value between dry eyes or blepharitis in the case of overexpression and dividing it by the control group value, or, in the case of repression, with a negative symbol, the control value divided by the lowest value between dry eye or blepharitis.

Once the proteins which, according to the proteomics studies, are capable of differentiating better between the different groups (Annexin A1, Cystatin S, Cystatin N, Glutathione S-transferase P, S100A4 and S100A6) have been identified by means of mass spectrometry, a cluster of a total of 19 candidate proteins for the subsequent validation studies has been selected by means of using bioinformatics tools for studying expression and functional networks.

These validation analyses include the candidate markers resulting from the differential expression analysis, together with those proteins which are directly or indirectly related through expression and functional networks, as well as those markers described in the literature for the pathologies analyzed in this study.

### EXAMPLE 3: Biological functionality study

The principal component analysis (PCA) performed from the raw data of expression of those spots which are statistically significant assures a perfect separation between the different groups, as can be seen in Figure 7. This means that the selected spots are potential biomarkers, so they have been identified by means of MALDI-TOF/TOF mass spectrometry. Once the marker proteins have been identified by mass spectrometry, a study of their biological functionality was performed. Thus, the inventors verified that a high percentage of the candidates to new markers were compromised in functions related to inflammation, immune response and to oxidative stress, as illustrated in Figure 8, biological processes which are closely related to the dry eyes pathology.

Once the expression and functional networks formed by the 7 previously identified markers have been analyzed, and observing that the biological processes most involved were the inflammatory and oxidative stress processes, 10 new markers related to these processes were incorporated to the group of markers for validation, these new markers being: PLAA, GSTM 2, GSTA 4, SSB, Annexin 11, Serpin B3, RCN1, SOD2, SMAD3 and JNK.

Table 5 shows a list with the names of the candidates to markers for the dry eyes and blepharitis pathologies, classified into three groups (Groups 1, 2 and 3), according to way in which they are obtained. Group 1 includes the markers obtained from the differential expression studies by means of 2-D SDS-PAGE, Group 2 includes those obtained by expression network analysis and functional enrichment analysis and Group 3 includes those previously reported in the literature and validated by Western blot.

**Table 5: List of markers for dry eye and blepharitis pathologies**

| **Gene** | **Protein** | **UniProt/ SwissProt** |
|---|---|---|
| **GROUP 1** | | |
| **CST4** | Cystatin S = Cystatin 4 | P01036 |
| **CST1** | Cystatin N = Cystatin 1 | P01037 |
| **S100A6** | S100 calcium-binding protein A6 | P06703 |
| **S100A4** | S100 calcium-binding protein A4 | P06702 |
| **GSTP1** | Glutathione S-transferase P | P09211 |
| **LGALS7** | Galectin 7 | P47929 |
| **ANX A1** | Annexin 1 | P04083 |
| **GROUP 2** | | |
| **Serpin B13** | Serpin B13 | Q9UIV8 |
| **RCN 1** | Reticulocalbin 1 | Q15293 |
| **SOD2** | Superoxide dismutase | P04179 |
| **SMAD3** | Mothers against decapentaplegic homolog 3 | P84022 |
| **JNK** | Stress-activated protein kinase= MAPK8 | P45983 |
| **PLAA** | Phosp. A2 activator protein | Q9Y263 |
| **GSTM 2** | Glutathione S-transferase Mu 2 | P28161 |
| **GSTA 4** | Glutathione S-transferase A4 | 015217 |
| **SSB** | Lupus La protein, SS type B antigen | P05455 |
| **ANX A11** | Annexin 11 | P50995 |
| **GROUP 3** | | |
| **SG1D1** | Secretoglobin family 1D member 1 (Lipophilin A) | 095968 |
| **PRDX5** | Peroxiredoxin 5 | P30044 |

### EXAMPLE 4: Validation of JNK and GAL-7 by means of sandwich ELISA

Validation studies for the JNK and GAL-7 proteins have been performed by means of Sandwich type ELISA, adding 5 µg of total protein in each of the cases. The quantification of both ELISA systems is based on an indirect detection of the antigens. Biotinylated primary anti-JNK or anti-GAL-7 antibodies were added first. A second incubation was subsequently performed with horseradish peroxidase (HRP)-conjugated streptavidin and finally tetramethylbenzidine (TMB) for the development and 1 M sulfuric acid, for the purpose of stopping the reaction. The signal of the wells was detected by means of measuring the absorbance at 450 nm. Both ELISA kits incorporated a calibration line in the range between 3333 pg/ml and 13.72 pg/ml with a minimum sensitivity of 20 pg/ml. Once the results of both assays were obtained, it was verified that the variations between the groups in the case of GAL-7 were significant. The statistical analysis performed for the concentration values obtained for this protein indicates, for Dry Eyes versus the reference sample, sensitivity and specificity values for this marker of 100% and 91.7 respectively, and an area under the ROC curve (AUC) of 0.988, as shown in Figure 9, the quantification of this marker indicating an excellent precision as a diagnostic methodology. Likewise, Figure 10 indicates, in the case of Blepharitis versus the reference sample, that the critical point of Galectin 7 concentration is 1790 pg/ml, which indicates that 91.7% specificity and 75% sensitivity are achieved below said value.

In addition, the validation of one of the selected candidate markers, JNK, has been performed by means of functional network analysis. In addition to the fact that this protein has been recently described as a possible marker for dry eyes, in the ELISA assays carried out for the JNK protein, an increase in the expression was observed in the case of the individuals with dry eyes. However, the concentration values obtained are very close to the limit of detection, so a broader evaluation with tear samples from new patients using a larger amount of protein for the determination of its concentration in tears is necessary.

## Claims

1. An *in vitro* method for diagnosing dry eye in a subject which comprises:
(i) determining the level of one or more proteins selected from the group of Lipophilin A, Peroxiredoxin 5, Cystatin S, Cystatin N, S100A4, S100A6, GST P1, Annexin 1 and Galectin 7 or a functionally equivalent variant thereof, in a sample from a subject; and
(ii) comparing the level of said protein or proteins with the level of said protein or proteins in a reference sample,
wherein if the subject has dry eye, the level of at least one of the S100A4, S100A6, GST P1 and Annexin 1 proteins is elevated with respect to its level in the reference sample and/or the level of at least one of the Cystatin S, Cystatin N, Galectin 7, Lipophilin A and Peroxiredoxin 5 proteins is reduced with respect to its level in the reference sample.

2. The method according to claim 1, wherein the level of one or more proteins selected from the group of Annexin 11, Serpin B13, Reticulocalbin 1, Superoxide dismutase, SMAD3, JNK, PLAA, GSTM2, GSTA4 and SSB or a functionally equivalent variant thereof is additionally determined.

3. An *in vitro* method for diagnosing blepharitis in a subject which comprises:
(i) determining the level of one or more proteins selected from the group of Lipophilin A, Peroxiredoxin 5, Cystatin S, Cystatin N, S100A4, S100A6, GST P1, Annexin 1 and Galectin 7 or a functionally equivalent variant thereof, in a sample from a subject; and
(ii) comparing the level of said protein or proteins with the level of said protein or proteins in a reference sample,
wherein if the subject has blepharitis, the level of at least one of the GST P1 and Annexin 1 proteins is elevated with respect to its level in the reference sample, the level of the S100A4 protein remains identical with respect to its level in the reference sample and/or the level of at least one of the Cystatin S, Cystatin N, S100A6, Galectin 7, Lipophilin A and Peroxiredoxin 5 proteins is reduced with respect to its level in the reference sample.

4. The method according to claim 3, wherein the level of one or more proteins selected from the group of Annexin 11, Serpin B13, Reticulocalbin 1, Superoxide dismutase, SMAD3, JNK, PLAA, GSTM2, GSTA4 and SSB or a functional variant thereof is additionally determined.

5. An *in vitro* method for differentiating between dry eye and blepharitis in a subject which comprises:
(i) determining the level of one or more proteins selected from the group of Lipophilin A, Peroxiredoxin 5, Cystatin S, Cystatin N, S100A4, S100A6, GST P1, Annexin 1 and Galectin 7 or a functionally equivalent variant thereof, in a sample from a subject; and
(ii) comparing the level of said protein or proteins with the level of said protein or proteins in a reference sample.

6. The method according to claim 5, wherein the level of one or more proteins selected from the group of Annexin 11, Serpin B13, Reticulocalbin 1, Superoxide dismutase, SMAD3, JNK, PLAA, GSTM2, GSTA4 and SSB is additionally determined.

7. The method according to any of the previous claims, wherein the sample is a tear sample from the subject.

8. An *in vitro* method for diagnosing blepharitis or dry eye in a tear sample from a subject which comprises:
(i) determining the level of Galectin 7 or a functionally equivalent variant thereof in said sample; and
(ii) comparing the level of said protein with its level in a reference sample.

9. A kit comprising at least one reagent for the detection of at least one protein selected from the group of Lipophilin A, Peroxiredoxin 5, Cystatin S, Cystatin N, S100A4, S100A6, GST P1, Annexin 1 and Galectin 7 or a functional variant thereof.

10. The kit according to claim 9, additionally comprising reagents for the detection of one or more proteins selected from the group of Annexin 11, Serpin B13, Reticulocalbin 1, Superoxide dismutase, SMAD3, JNK, PLAA, GSTM2, GSTA4 and SSB.

11. The kit according to claim 10, further comprising a positive control.

12. The kit according to any of claims 9 to 11 for its use in the *in vitro* diagnosis of dry eye or blepharitis.
